(19) **Europäisches Patentamt / European Patent Office / Office européen des brevets**

(11) **EP 2 074 938 B1**

(12) **EUROPEAN PATENT SPECIFICATION**

(45) Date of publication and mention
of the grant of the patent:
**09.02.2011 Bulletin 2011/06**

(51) Int Cl.:
***A61B 5/00*** *(2006.01)*

(21) Application number: **08022265.6**

(22) Date of filing: **22.12.2008**

(54) **A noninvasive living body measuring device and a noninvasive living body measuring method**

Vorrichtung und Verfahren zur nichtinvasiven Messung eines lebenden Körpers

Dispositif non invasif de mesure d'organisme vivant et procédé non invasif de mesure d'organisme vivant

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.12.2007 JP 2007336392**

(43) Date of publication of application:
**01.07.2009 Bulletin 2009/27**

(73) Proprietor: **SYSMEX CORPORATION**
**Kobe-shi,**
**Hyogo 651-0073 (JP)**

(72) Inventors:
• **Ozawa, Toshiyuki**
**Kobe-shi**
**Hyogo 651-0073 (JP)**

• **Ohnishi, Yasuhito**
**Kobe-shi**
**Hyogo 651-0073 (JP)**

(74) Representative: **HOFFMANN EITLE**
**Patent- und Rechtsanwälte**
**Arabellastraße 4**
**81925 München (DE)**

(56) References cited:
**EP-A- 1 040 788     EP-A- 1 447 044**
**EP-A- 1 743 570     WO-A-2004/107971**
**WO-A-2006/131881**

**Description**

FIELD OF THE INVENTION

The present invention relates to a noninvasive living body measuring device and a noninvasive living body measuring method.

BACKGROUND

In the past, for example, a noninvasive living body measuring device disclosed in US Patent No. 7280860 has been known as a device for measuring components of a blood by imaging a living body with the use of image pick-up means and analyzing a blood vessel in a living body image. The noninvasive living body measuring device measures blood components from the image obtained by illuminating a wrist including a blood vessel (vein) with a light source and imaging the illuminated wrist.

The noninvasive living body measuring device includes a detection section which is mounted on an arm of a person and an analysis section which is connected to the detection section. The detection section includes a support base, a rotary base which is vertically inserted into a central opening of the support base so as to be rotatably supported, a housing which is mounted on a central opening of the rotary base, and a pair of holding pieces for fixing the support base to a wrist. The support base has a circular opening at the bottom thereof, and a light source section composed of 6 light emitting diodes disposed concentrically with the opening is provided around the opening. In the housing, an image pick-up section is housed to image an image pick-up area on a living body illuminated by the light source section.

When the noninvasive living body measuring device performs a measurement operation, the detection section is mounted on the wrist, and the housing is rotated to adjust a position of the image pick-up area while a monitor image of an output section provided in the analysis section is observed.

However, the device described in US Patent No. 7280860 is constituted so that the housing including the image pick-up section can be rotated but a position of the image pick-up section is determined by the position in which the detection section is fixed to the wrist. Consequently, once the detection section is fixed to the arm, the movement of only the image pick-up section cannot be performed. Accordingly, for example, when the image pick-up section is not disposed at a position in which a measurement target blood vessel can be imaged, it is required to move the image pick-up section together with a body of the device to change the position of the image pick-up section and the operation thereof is complicated.

In addition to the above, EP 1 743 570 A1 discloses a non-invasive living body measuring device in which said device is positioned on a blood vessel for capturing an image thereof. It is further taught to provide the device with lighted markers that instruct a user to move or rotate the entire device body in order to correctly position an imaging part to a region suited for imaging a blood vessel. However, the entire device body must be moved or rotated in order to adjust for a suitable imaging region. In this way, the respective disclosure is not concerned with an image pick-up section position adjusting section adjusting the position of an image pick-up section relative to a device body that is fixed to a living body.

Further, also EP 1 447 044 A1 is concerned with a non-invasive living body measuring device.

SUMMARY OF THE INVENTION

In a first aspect of the present invention, there is provided a noninvasive living body measuring device comprising: an image pick-up section for imaging a living body; a device body comprising an analysis section acquiring biological information by analyzing an image obtained by an image pick-up operation of the image pick-up section; a fixing section for fixing the device body to a living body; and an image pick-up section position adjusting section for adjusting a position of the image pick-up section on a living body in a state in which the device body is fixed to a living body, wherein the image pick-up section position adjusting section adjusts the position of the image pick-up section relative to the device body by a rotational movement of the image pick-up section relative to the device body.

In a second aspect of the present invention, there is provided a noninvasive living body measuring method comprising the steps of: fixing to an arm a device body including an image pick-up section for imaging a living body and an analysis section for acquiring biological information by analyzing an image obtained by the image pick-up operation of the image pick-up section; adjusting a position of the image pick-up section on palmar side of a wrist of the arm relative to the device body by a rotational movement of the image pick-up section relative to the device body; imaging a site of the living body corresponding to the position adjusted in the adjusting step by the image pick-up section; and acquiring a biological information by analyzing with the analysis section an image obtained in the imaging step.

## BRIEF DESCRIPTION OF THE DRAWINGS

Fig. 1 is a perspective view showing the appearance of a device according to this embodiment;
Fig. 2 is an exploded perspective view of the device according to this embodiment;
Fig. 3 is a plan view showing the configuration of a light source section;
Fig. 4 is a diagram showing a position relationship of 4 light emitting diodes provided in a holding plate;
Fig. 5 is a block diagram showing the configuration of a control section;
Figs. 6A to 6C are diagrams showing a state in which an image pick-up section rotating section is rotated;
Fig. 7 is a cross-sectional view schematically showing the configuration of an image pick-up section position adjusting section;
Figs. 8A to 8C are diagrams showing a state in which a receiving section is moved by the image pick-up section position adjusting section;
Fig. 9 is a perspective view showing an example of a usage state of the device according to this embodiment;
Fig. 10 is a diagram showing a state in which the device is mounted on an arm;
Fig. 11 is a flowchart showing the measurement operation of the device;
Fig. 12 is a diagram showing an example of a screen displayed when the device is in a standby state;
Fig. 13 is a diagram showing a rectangular area including an image pick-up area CR, which is coordinate-divided in a two-dimensional x-y coordinate where $0 \leq x \leq 640$ and $0 \leq y \leq 480$;
Fig. 14 is a diagram showing an example of a luminance profile (luminance profile PF) of a pixel in an x direction in a predetermined y coordinate;
Fig. 15 is an explanatory diagram showing a method of obtaining a position of a blood vessel;
Fig. 16 is a diagram showing an example of a positioning screen of the blood vessel;
Fig. 17 is a flowchart showing in detail a process of indicating the direction in which the image pick-up section should be moved, which is performed in Step S16 of the flowchart shown in Fig. 11;
Figs. 18A to 18C are diagrams showing an example of a display screen of a display section 8 when a movement direction of the image pick-up section is instructed;
Fig. 19 is a diagram showing an example of a screen when the device 1 completes the measurement;
Fig. 20 is a flowchart showing in detail a hemoglobin concentration measuring process performed in Step S20 of the flowchart shown in Fig. 11;
Fig. 21 is a diagram showing the distribution of a luminance B at a position X;
Fig. 22 is a diagram showing the distribution of a concentration D at a position X;
Fig. 23 is a diagram showing the distribution of the concentration D at the position X;
Fig. 24 is a graph plotting the calculated values of the device according to this embodiment and actual measured values obtained from a hemocytometer or the like for hemoglobin concentrations of a plurality of subjects; and
Fig. 25 is a flowchart showing another example of the measurement operation of the device.

## DETAILED DESCRIPTION OF THE PREFERRED EMBODIMENTS

Hereinafter, embodiments of a sample analyzer of the invention will be described in detail with reference to the accompanying drawings.

Hereinafter, embodiments of a noninvasive living body measuring device according to the invention will be described in detail with reference to the accompanying drawings.

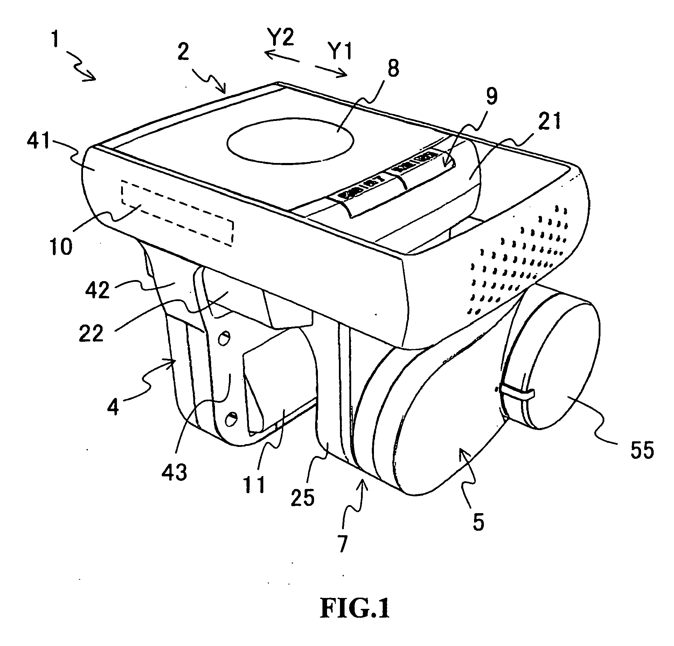

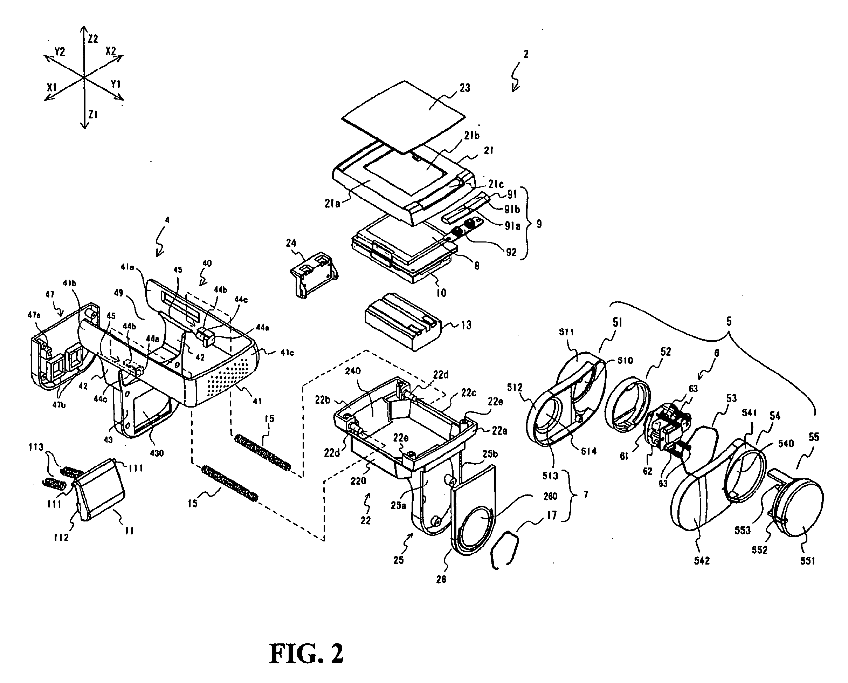

Fig. 1 is a perspective view showing the appearance of the noninvasive living body measuring device (hereinafter, referred to as the device 1) according to the invention. The device 1 is a blood component measuring device which is mounted on an arm of a user to image a blood vessel existing from the arm to a wrist and to measure hemoglobin concentration on the basis of a picked-up image. The device 1 mainly includes a device body 2, a slide member 4, a receiving section 5, an image pick-up section 6 (see Fig. 2), an image pick-up section position adjusting section 7, a display section 8, an operation section 9, a control section 10, and a separation preventing member 11.

The device body 2 includes the control section 10 for analyzing a living body image picked up by the image pick-up section 6, the display section 8 for displaying the living body image picked up by the image pick-up section 6, an index indicating the position adjustment of the image pick-up section 6, a measurement result or the like, and the operation section 9 for operating the device 1.

Further, the device body 2 is provided with a first fixing member 25. The first fixing member 25 holds the arm of the user with a second fixing member 43 provided in the slide member 4 to fix the device body 2 to the arm. The receiving section 5 is connected to the first fixing member 25 via the image pick-up section position adjusting section 7 and the image pick-up section 6 is received in the receiving section 5. The receiving section 5 is provided with an image pick-up section rotating section 55 for rotating the image pick-up section 6.

The slide member 4 includes a frame 41 which is provided so as to surround a side of the device body 2, a connection section 42, and the second fixing member 43 which is connected to the frame 41 by the connection section 42, and is supported slidably in a Y1 direction and a Y2 direction by the device body 2. The second fixing member 43 is provided with the separation preventing member 11 for preventing the separation of the arm of the user when the device body 2 is fixed to the arm.

Components of Device 1

Fig. 2 is an exploded perspective view of the noninvasive living body measuring device according to this embodiment. Hereinafter, components of the noninvasive living body measuring device will be described with reference to Fig. 2. Next, the combination of the components will be described.

The device body 2 includes a liquid crystal cover 23, an upper housing 21, a battery cover 24, a lower housing 22, the display section 8, the operation section 9 including an operation key 91 and a switch substrate 92, the control section 10, and a battery 13.

The liquid crystal cover 23 includes a transparent member having a substantially square shape and is used to protect .a display plane of the display section 8 received in the device body 2.

The upper housing 21 is a member which is attached to the lower housing 22 to constitute a housing of the device body 2. The upper surface of the upper housing 21 is provided with a depressed section 21a to which the liquid crystal cover 23 is fitted. At the center of the depressed section 21a, a first opening 21b corresponding to the display plane of the display section 8 attached to the lower side is provided. In addition, near the depressed section 21a, a second opening 21c to which the operation key 91 and the switch substrate 92 are fitted is provided.

The operation section 9 includes the operation key 91 and the switch substrate 92. The operation key 91 includes a "start/completion" key 91a and "measurement/record" key 91b and is electrically connected to the switch substrate 92 corresponding to the keys. The switch substrate 92 is further electrically connected to the control section 10 to be described later.

The display section 8 includes a liquid crystal panel and displays a blood vessel image or a measurement result on the basis of an image signal output from the control section 10. The display is switched in accordance with the state of the device 1. For example, screens corresponding to a standby state, a blood vessel position adjustment moment, and the completion of measurement are displayed on the display section 8.

The control section 10 is composed of an analog substrate and a digital substrate. A CPU 100a to be described later controls an operation of each section. The configuration of the control section 10 will be described in detail later.

The battery 13 includes a lithium ion secondary battery and a secondary battery including a nickel-cadmium rechargeable battery or the like and supplies electric power to the display section 8, the control section 10 and the image pick-up section 6.

The lower housing 22 includes a front chassis section 22a, a back chassis section 22b, side end sections 22c for connecting the front chassis section 22a and the back chassis section 22b, a box-shaped battery receiving section 220 connected to the lower sides of the side end sections 22c and a first fixing member 25 attached to the lower side of the front chassis section 22a.

Both ends of the back chassis section 22b are bent inwardly and provided with a spring supporter 22d horizontally extending in the Y1 direction. The spring supporter 22d is formed in a cylindrical shape, and as described later, a spring 15 is mounted on the spring supporter 22d. The box-shaped battery receiving section 220 receives the battery 13. The back side of the battery receiving section 220 is provided with an opening 240 and a battery cover 24 is attached after the battery 13 is received in the battery receiving section 220.

The first fixing member 25 has a framework 25a and a back wall section 25b connected to the bottom section of the battery receiving section 220. A cover member 26 is attached to the front surface of the first fixing member 25. The cover member 26 includes a plate-shaped member of which the lower side is round.

The slide member 4 includes a slide member body 40 and a back surface cover member 47. The slide member body 40 includes a frame member 41 having left and right side wall members 41a and 41b and a connecting wall section 41c for connecting the left and right side wall members 41a and 41b, connecting sections 42, each of which extends downwardly from the left or right side wall member 41a or 41b via a step section 45, and a plate-shaped second fixing member 43 connected to the frame member 41 by the connecting sections 42. The second fixing member 43 is a plate-shaped member suspended with respect to the connecting sections 42 and has a rectangular window section 430 at the center thereof. A thin wall is formed on the back side of the second fixing member 43 via the step sections, and a thickness when the back surface cover member 47 is attached is the same as the width of the connecting section 42.

The inside of the left and right side wall members 41a and 41b of the frame member 41 is provided with a suspending piece section 44a, a horizontal piece section 44b extending in the Y2 direction from the upper end of the suspending piece section 44a, and a spring supporter 44c extending parallel to the side wall in the Y2 direction from a side surface of the suspending piece section 44a.

The back surface cover member 47 is a member having a shape and a size to be attachable to the thin wall formed on the back surface of the second fixing member 43. On the inside of the back surface cover member, bearing sections 47a for holding a spindle 111 provided in the separation preventing member 11 and a pair of spring receiving sections 47b for holding one ends of springs 113 are provided.

The separation preventing member 11 has a size to be fitted to the window section 430 provided in the second fixing member 43. The front wall section of the separation preventing member forms an inclined surface from the upper end to the lower end thereof. A side surface of the separation preventing member 11 is provided with the rod-shaped spindle 111 and an engaging member 112.

The receiving section 5 is a member for receiving the image pick-up section 6 and includes an inner housing 51, a turret 52, an elastic member 53, an outer housing 54 and the image pick-up section rotating section 55.

The inner housing 51 is provided with a cylindrical section 511 in which the image pick-up section 6 is received, an extending section 512 extending forwardly from the cylindrical section 511, a circular opening 514 provided in the extending section 512 and a cylindrical fitting section 513 provided at the opening edge of the opening 514. The turret 52 includes a cylindrical framework and is provided with, at the center thereof, a window section 520 to which an image pick-up surface of the image pick-up section 6 is fitted. The outer housing 54 includes a cylindrical section 541 provided to correspond to the cylindrical section 511 of the inner housing 51 an an extending section 542. The image pick-up section rotating section 55 includes a cylindrical substrate 551, a fitting section 552 having a smaller diameter than the substrate 551 and fitted to an opening 540 of the outer housing 54, and a plurality of spring supporters 553 provided inside the substrate 551 to support a plurality of compression springs 63 attached to the image pick-up section 6.

The image pick-up section 6 received in the receiving section 5 includes a light source section 61 for illuminating a living body and a CCD image pick-up element 62 for imaging a site of the living body illuminated by the light source section 61.

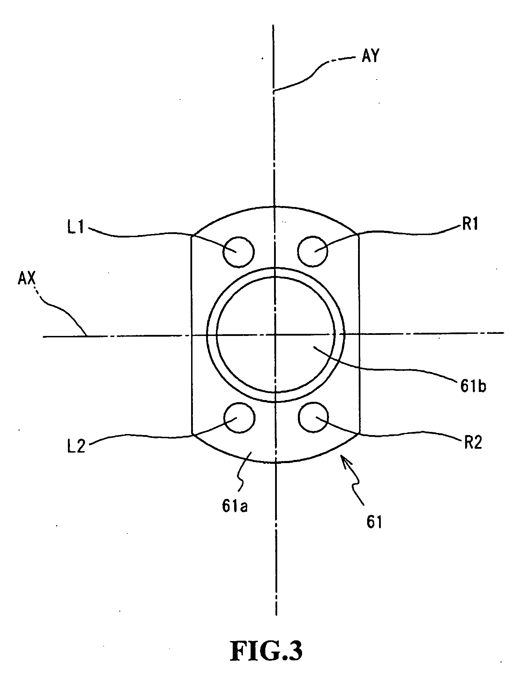

Fig. 3 is a plan view showing the configuration of the light source section 61. The light source section 61 includes a substantially rectangular holding plate 61a of which the short sides are round and 4 light emitting diodes R1, R2, L1 and L2 held in the holding plate 61a. At the center of the holding plate 61a, a circular opening 61b through which light incident to the CCD image pick-up element 62 passes is provided and the above-described light emitting diodes are disposed around the opening 61b.

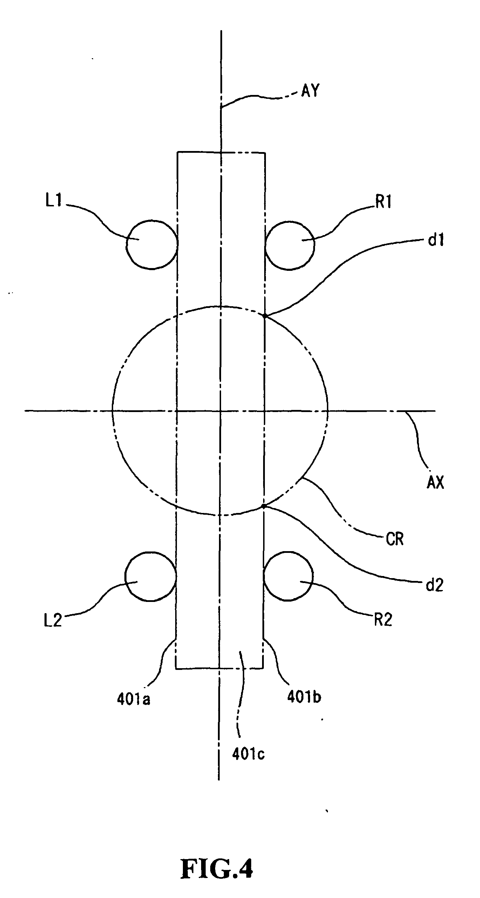

Fig. 4 is a diagram showing a position relationship of the 4 light emitting diodes provided in the holding plate 61a. The light emitting diodes R1, R2, L1 and L2 are respectively disposed symmetrically with respect to a first axis AY and a second axis AX which pass through the center of the opening 61b and are perpendicular to each other. In a state in which the device 1 is mounted on a wrist, an image pick-up area CR of a surface of the wrist is an area for being imaged by the CCD image pick-up element 62 to be displayed on the display section 8. An area 401c between an index line 401a on the side of the light emitting diodes L1 and L2 (second light emitting section) and an index line 401b on the side of the light emitting diodes R1 and R2 (first light emitting section) is the area suitable for the image pick-up operation of the CCD image pick-up element 62, that is, an area in which a blood vessel is to be positioned when the image pick-up operation is performed. The index lines 401a and 401b are displayed on the display section 8 by the control section 10, as described later. When blood component analysis is performed, a mounting position of the device body 2 is adjusted so as to position an arbitrary blood vessel of the wrist in the area 401c. The blood vessel is illuminated with near-infrared light (central wavelength = 805 nm) from both sides by the light emitting diodes R1, R2, L1 and L2.

The CCD image pick-up element 62 is disposed in back of the holding plate 61a so as to pick up an image of the image pick-up area CR. A lens (not shown) is provided between the opening 61b provided at the center of the holding plate 61a and the CCD image pick-up element 62, and the CCD image pick-up element 62 picks up a living body image formed via the lens and sends an image signal to the control section 10.

The image pick-up section position adjusting section 7 includes the cover member 26 attached to the first fixing member 25 of the device body 2, an elastic member 17 and the fitting section 513 provided in the extending section 512 of the inner housing 51.

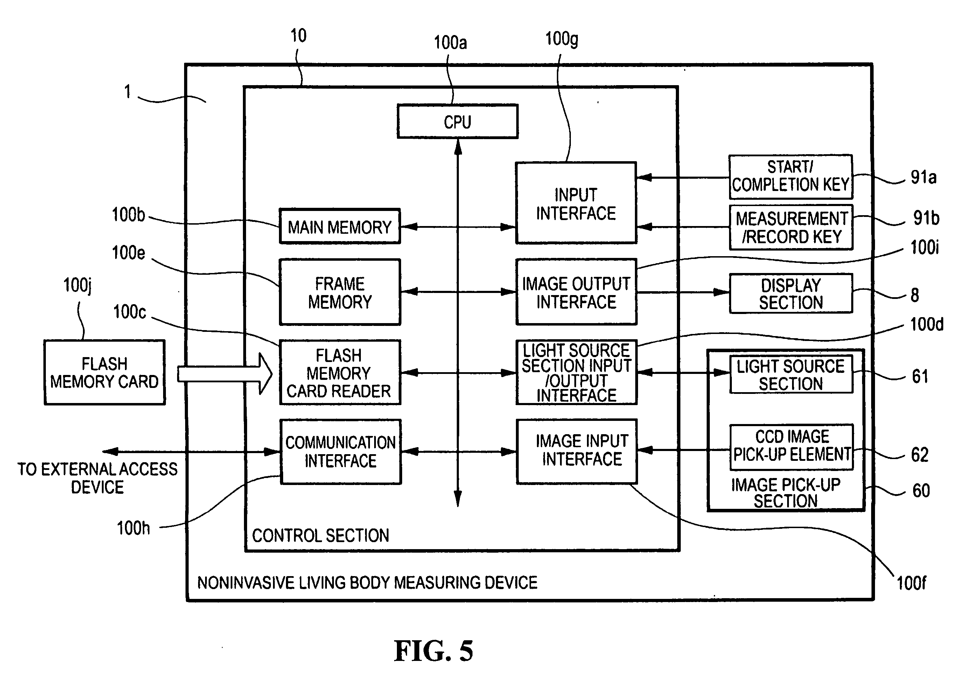

Next, the configuration of the control section 10 will be described. Fig. 5 is a block diagram showing the configuration of the control section 10. The control section 10 includes a CPU 100a, a main memory 100b, a flash memory card reader 100c, a light source section input/output interface 100d, a frame memory 100e, an image input interface 100f, an input interface 100g, a communication interface 100h and an image output interface 100i. The CPU 100a is connected to the main memory 100b, the flash memory card reader 100c, the light source section input/output interface 100d, the frame memory 100e, the image input interface 100f, the input interface 100g, the communication interface 100h and the image output interface 100i via a data transmission line so as to transmit data to each other. Thanks to this configuration, the CPU 100a can read/write data from/to the main memory 100b, the flash memory card reader 100c and the frame memory 100e and can transmit/receive the data to/from the light source section input/output interface 100d, the image input interface 100f, the input interface 100g, the image output interface 100i and the communication interface 100h.

The CPU 100a can execute a computer program loaded to the main memory 100b. By executing the computer program to be described later with the CPU 100a, the device functions as a noninvasive living body measuring device.

The main memory 100b includes a SRAM, a DRAM or the like. The main memory 100b is used to read computer programs stored in a flash memory card 100j. When the computer programs are executed, the main memory is used as a work area of the CPU 100a.

The flash memory card reader 100c is used to read data stored in the flash memory card 100j. The flash memory card 100j has a flash memory (not shown), and can hold data even when electric power is not supplied from the outside. In the flash memory card 100j, a computer program to be executed by the CPU 100a and data to be used for the execution operation are stored.

In addition, for example, an operating system based on TRON specifications is installed on the flash memory card 100j. The operating system is not limited thereto and may be, for example, an operating system providing a graphical user interface environment such as Windows (registered trade name) made and distributed by Microsoft corporation, America. In the following description, the computer program according to this embodiment operates on the operating system.

The light source section input/output interface 100d includes an analog interface including a D/A converter and an A/D converter. The light source section input/output interface 100d is electrically connected to each of the 4 light emitting diodes R1, R2, L1 and L2 provided in the light source section 61 by an electric signal line to control operations of the light emitting diodes. On the basis of a computer program to be described later, the light source section input/output interface 100d controls currents which are applied to the light emitting diodes R1, R2, L1 and L2.

The frame memory 100e includes a SRAM, a DRAM or the like. The frame memory 100e is used to store data when the image input interface 100f to be described later performs image processing.

The image input interface 100f includes a video digitize circuit (not shown) including an A/D converter. The image input interface 100f is electrically connected to the CCD image pick-up element 62 by an electric signal line and an image signal is input from the CCD image pick-up element 62. The image signal input from the CCD image pick-up element 62 is A/D converted by the image input interface 100f. The image data subjected to digital conversion in this manner is stored in the frame memory 100e.

The input interface 100g includes an analog interface including an A/D converter. "Start/completion" key 91a and "measurement/record" key 91b are electrically connected to the input interface 100g. Thanks to this configuration, the user can select operation items of the device by using the "measurement/record" key 91b, and can turn on/off the device and execute the operation selected by the "measurement/record" key 91b by using the "start/completion key 91a".

The communication interface 100h includes, for example, a serial interface such as USB, IEEE1394 or RS232C, or a parallel interface such as SCSI. The control section 10 uses a predetermined communication protocol by the communication interface 100h to transmit/receive data to/from an external access device such as a mobile computer or a cellular phone. In this manner, the control section 10 sends measurement result data to the external access device via the communication interface 100h.

The image output interface 100i is electrically connected to the display section 8 and outputs a video signal based on the image data applied from the CPU 100a to the display section 8.

Assembly of Device 1

The assembly of the device body 2 will be described with reference to Fig. 2. First, the display section 8 is fitted to the depressed section 21a provided in the upper surface of the upper housing 21. From the lower side, the operation key 91 is fitted to the opening 21c provided in the upper surface of the upper housing 21. In addition, the switch substrate 92 is connected to the lower part of the operation key 91. Next, the display section 8 and the control section 10 are attached to the upper housing 21.

Next, the lower housing 22 is inserted into a hollow section 49 surrounded by the connecting sections 42 of the slide member 4 so that one ends of the springs 15 are each mounted on the spring supporter 22d provided in the lower housing 22 and the other ends are each mounted on the spring supporter 44c provided inside the left or right side wall member 41a or 41b of the slide member 4, as shown by the dotted lines of Fig. 2. At this time, the side end sections 22c of the lower housing 22 are each disposed at the step section 45 provided between the left or right side wall member 41a or 41b and the connecting section 42.

The springs 15 supported by the lower housing 22 and the slide member 4 apply a pressing force to the lower housing 22 and the slide member 4. The lower housing 22 is pressed against the slide member 4 in the Y2 direction and the slide member 4 is pressed against the lower housing 22 in the Y1 direction. At this time, when ends 22e of the front chassis section 22a of the lower housing 22 are each brought into contact with the suspending piece section 44a of the slide member 4, the movement of the lower housing 22 pressed in the Y2 direction is stopped at a certain position. Further, the horizontal piece sections 44b provided inside the left and right side wall members 41a and 41b prevent the springs 15 attached to the spring supporters 44c from being upwardly separated. In addition, when the side end sections 22c of the lower housing 22 are disposed at the step sections 45 provided inside the left and right side wall members 41a and 41b, the separation of the lower housing 22 is prevented and the slide movement of the lower housing 22 in the Y1 and Y2 directions is guided.

In a state in which the lower housing 22 and the slide member 4 are connected to each other by the springs 15, the upper housing 21 to which the liquid crystal cover 23, the operation key 91, the switch substrate 92, the display section 8, and the control section 10 are attached is mounted and the upper housing 21 and the lower housing 22 are fixed by screws inserted from the lower side of the lower housing 22. The battery 13 is received in the battery receiving section 220 of the lower housing 22 via the opening 240 and the opening 240 is closed by the battery cover 24.

Next, the separation preventing member 11 and the back surface cover member 47 are attached to the slide member 4. First, one ends of the springs 113 are each held by a spring holding section provided in the back surface of the separation preventing member 11 and the other ends are each held by the spring receiving section 47b of the back surface cover member 47. The spindles 111 provided in the side surfaces of the separation preventing member 11 engage with the bearing sections 47a of the back surface cover member 47, respectively. In addition, the separation preventing member 11 is fitted to the window section 430 of the second fixing member 43 and the back surface cover member 47 is attached to the second fixing member 43. The back surface cover member 47 is attached to the thin wall provided on the back side of the second fixing member 43 and the second fixing member 43 and the back surface cover member 47 are fixed by screws. At this time, the lower side of the separating preventing member 11 is pressed toward the second fixing member 43 by a pair of the springs 113. However, since the engaging member 112 provided in the side wall of the separation preventing member 11 engages with the outer edge of the window section 430, the separation preventing member 11 is held in a state in which the lower end thereof protrudes from the window section 430. In addition, the separation preventing member 11 can be freely taken out and put in by an elastic force of the springs 113.

Next, the combination of the receiving section 5 will be described. First, the turret 52 is inserted into a cylindrical opening 510 of the inner housings 51 to fit the holding plate 61a of the image pick-up section 6 to the window section 520 of the turret 52. Next, the outer housing 54 is fixed to the inner housing 51 by screws and the image pick-up section rotating section 55 is inserted into the cylindrical opening of the outer housing 54. At this time, the plurality of spring supporters 553 provided in the image pick-up section rotating section 55 are mounted on the plurality of compression springs 63 provided in the image pick-up section 6, and the fitting section 552 of the image pick-up section rotating section 55, around which the elastic member 53 is wound, is inserted into the outer housing 54. During the rotation of the image pick-up section rotating section 55, the elastic member 53 applies a suitable amount of a frictional force to a surface between the fitting section 552 and the outer housing 54 and functions as coming-off preventing means for the image pick-up section rotating section 55. By connecting the image pick-up section rotating section 55 to the image pick-up section 6 via the compression springs 63, the image pick-up section rotating section 55 and the image pick-up section 6 can integrally rotate. Further, by an elastic force of the compression springs 63, the image pick-up surface of the image pick-up section 6 presses a surface of a living body by a proper amount of pressure when the image pick-up surface of the image pick-up section 6 is brought into contact with the living body.

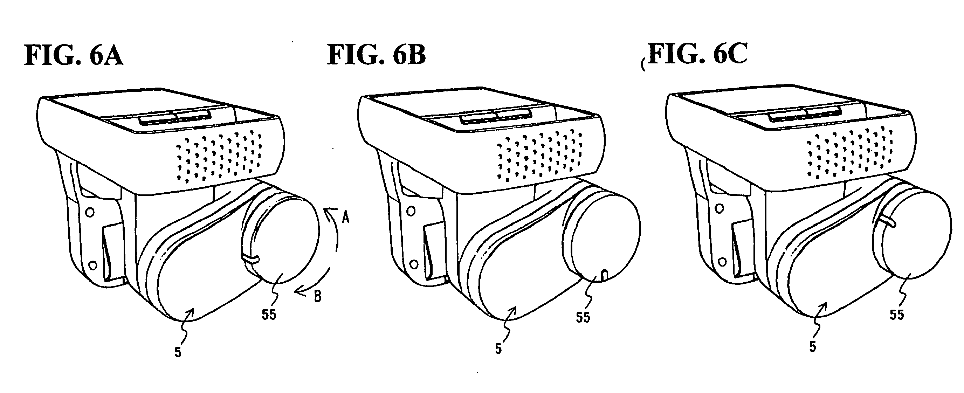

Figs. 6A, 6B and 6C are diagrams showing a state in which the image pick-up section rotating section 55 is rotated. The states in which the image pick-up section 6 is rotated in an A direction and a B direction from the state shown in Fig. 6A are shown in Figs. 6B and 6C, respectively. In this manner, the image pick-up section 6 can be rotated around the center of the opening 510 of the inner housing 51 in the receiving section 5. The user can perform the position adjustment operation by rotating the image pick-up section 6 so as to position a measurement target blood vessel in the area 401c (see Fig. 4) suitable for the image pick-up operation of the CCD image pick-up element 62.

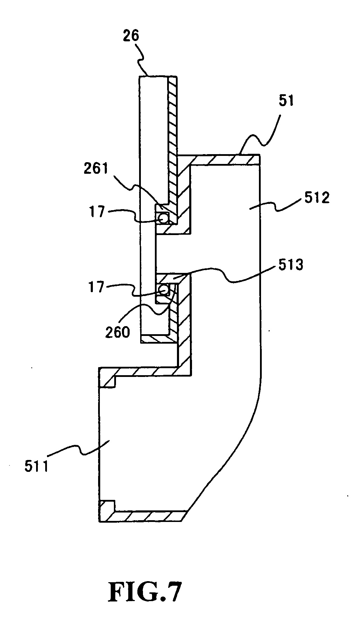

Next, the connection of the device body 2 and the receiving section 5 will be described using Fig. 7. Fig. 7 is a cross-sectional view schematically showing the configuration of the image pick-up section position adjusting section 7. The cover member 26 includes an opening 260 and a circular convex section 261 provided around the opening 260 and the fitting section 513 of the inner housing 51 is inserted into the opening 260. After the fitting section 513 is inserted into the opening 260, the elastic member 17 is attached to the fitting section 513. By attaching the elastic member 17 to the fitting section 513, the elastic member 17 applies a suitable amount of a frictional force to a surface between the fitting section 513 and circular convex section 261 during the rotation of the receiving section 5 and functions as coming-off preventing means for the fitting section 513. In addition, the cover member 26 is attached to the front surface of the first fixing member 25 and thus the device body 2 and the receiving section 5 are connected to each other.

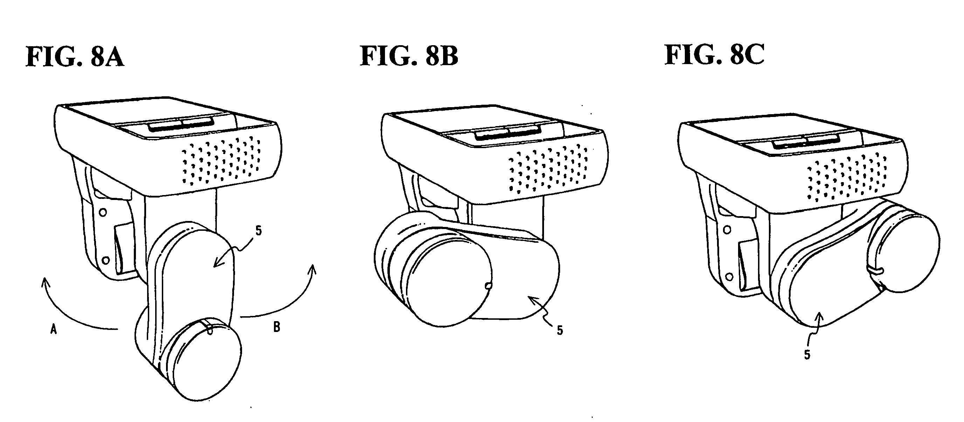

Figs. 8A, 8B and 8C are diagrams showing a state in which the receiving section 5 is moved by the image pick-up section position adjusting section 7. The states in which the receiving section 5 is moved in an A direction and a B direction from the state shown in Fig. 8A are shown in Figs. 8B and 8C, respectively. In this manner, the receiving section 5 holding the image pick-up section 6 can be rotated by about 180 degrees around the center of the opening 260 provided in the cover member 26. After mounting the device 1 on the arm, the user can easily adjust the position of the image pick-up section 6 without moving the device 1 itself when, for example, the measurement target blood vessel is not positioned in the image pick-up area CR (see Fig. 4) for the image pick-up section 6 or when a site of the image pick-up section 6, which is brought into contact with the living body, is to be finely adjusted.

Mounting of Device 1

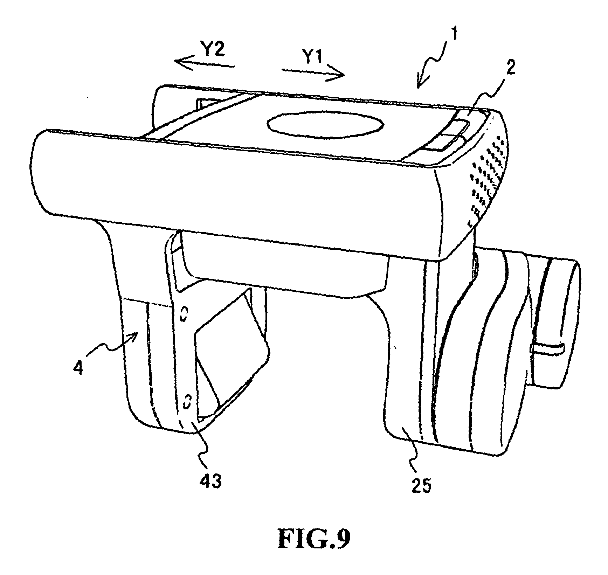

The device 1 having such a configuration is mounted on the wrist of the user as follows. First, when the connecting wall section 41c of the slide member 4 and the device body 2 are grasped by hand from the state shown in Fig. 1, the slide member 4 slides in the Y2 direction with respect to the device body 2 and the interval between the first fixing member 25 and the second fixing member 43 is simultaneously widened. The device 1 in this state is shown in Fig. 9.

The user inserts the arm between the first fixing member 25 and the second fixing member 43 while grasping the connecting wall section 41c of the slide member 4 and the back chassis section 22b of the device body 2 and then releases the grasped connecting wall section 41c and device body 2. When a grasping force disappears, the springs 15 connecting the device body 2 and the slide member 4 try to return to their original state by their own elastic force and thus the device body 2 is pushed in the Y1 direction and the slide member 4 is pushed in the Y2 direction. In this manner, the interval between the first fixing member 25 and the second fixing member 43 is narrowed, the arm inserted between the first fixing member 25 and the second fixing member 43 is elastically pressed and caught therebetween, and thus the device 1 is fixed to the arm.

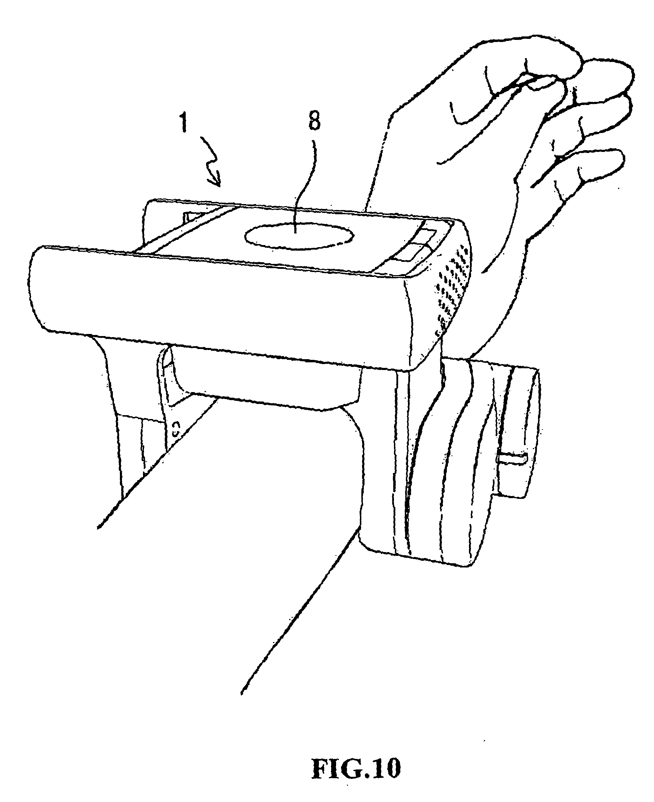

Fig. 10 is a diagram showing a state in which the device 1 is mounted on the arm. When the device 1 is mounted on the arm, the image pick-up section 6 is required to be brought into contact with the wrist which is an image pick-up site. Accordingly, as shown in Fig. 10, the device is mounted by catching the palmar side and the back side of the hand so that the first fixing member 25 holding the image pick-up section 6 is disposed on the palmar side.

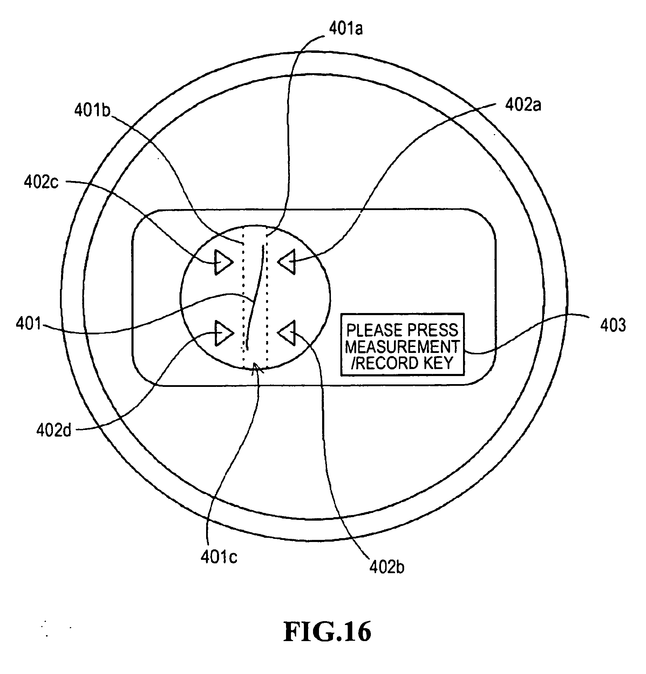

As is obvious from Fig. 10, when the device 1 is mounted on one arm (left arm in Fig. 10) so as to bring the image pick-up section 6 into contact with the wrist and the palm faces the other arm (right arm) in a state in which an elbow is bent by 90 degrees, the display screen of the display section 8 looks upward. In this manner, the user can adjust the position of the image pick-up section 6 while confirming the positioning screen (see Fig. 16) displayed on the display screen of the display section 8. At this time, the separation preventing member 11 protrudes in a state in which the lower side thereof is elastically pressed. Accordingly, the separation preventing member 11 functions so as to support the arm caught between the first individual member 3 and the second fixing member 43 from below and the separation of the device 1 from the arm is prevented.

Consequently, the device 1 according to this embodiment can be mounted on the arm only by the operation of grasping the connecting wall section 41c and the device body 2 with one hand and separating them from each other and the mounting on the arm is very easy.

Further, the distance between the first fixing member 25 and the second fixing member 43 is adjustable and thus it is possible to deal with even arm thicknesses different from one user to the next. Generally, the difference in arm thickness (thickness between palmar side and back side of hand of arm) among individuals is smaller than the difference in arm width (length in direction, perpendicular to arm thickness direction). Like the device 1 according to this embodiment, thanks to the configuration in which the arm is fixed by being caught in the thickness direction, it is possible to widely deal with the arm thicknesses different from one user to the next. In addition, thanks to the configuration in which the palmar side and the back side of the hand of the arm are caught, the contact area between the first and second fixing members 25 and 43 and the arm can be largely ensured and the device 1 becomes difficult to separate from the arm.

Since the arm of the user is pressed by a predetermined pressure with the use of the first fixing member 25 and the second fixing member 43, the blood flow in the vicinity of the wrist which is an image pick-up site is blocked and the blood vessel of the wrist expands. Accordingly, the device 1 functions as a pressure belt and thus a good blood vessel image can be picked up and hemoglobin concentration can be measured without the use of a pressure belt.

Fig. 10 shows the state in which the device 1 is mounted on the left arm, but may be mounted on the right arm. As described above, since the receiving section 5 can be rotated by 180 degrees by the image pick-up section position adjusting section 7 (see Fig. 8), the receiving section 5 which is in the state shown in Fig. 10 may be rotated by 180 degrees and the device 1 may be mounted on the right arm so as to bring the image pick-up section 6 into contact with the wrist. In this manner, by the image pick-up section position adjusting section 7, the device 1 according to this embodiment can be mounted on the left arm or the right arm and thus the device is easily mounted even when the user is right-handed or left-handed.

Measurement Operation of Device 1

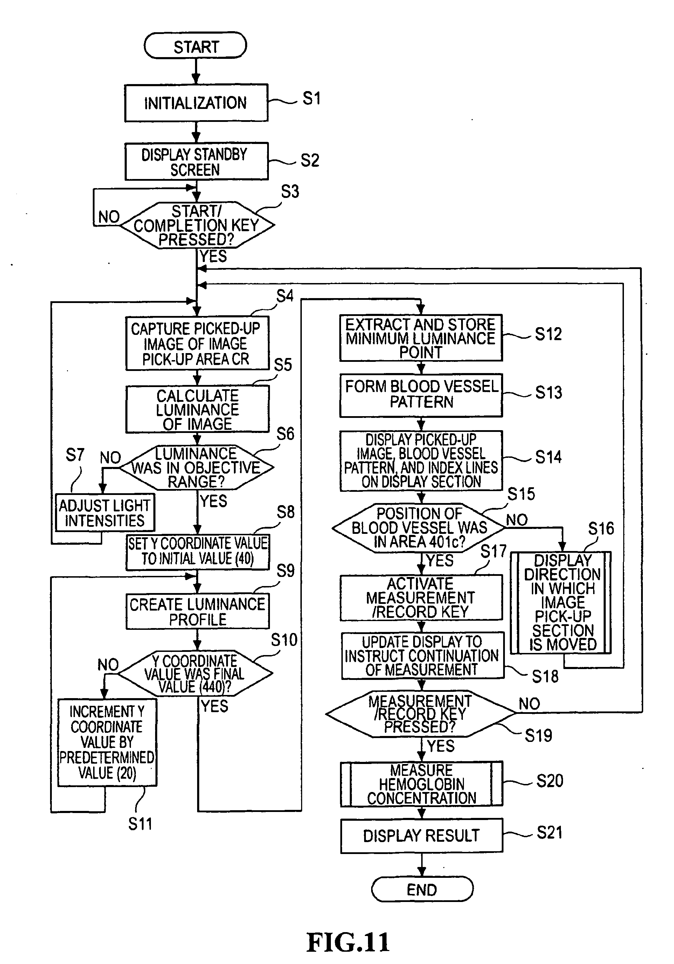

Next, the measurement operation of the device 1 will be described. Fig. 11 is a flowchart showing the measurement operation of the device 1. First, as shown in Fig. 10, the device 1 is mounted on the arm of the user and the position of the image pick-up section 6 is roughly adjusted by the image pick-up section position adjusting section 7 so as to bring the image pick-up section 6 into contact with the wrist. Next, when the user turns on the device 1 by pressing the "start/completion" key 91a provided in the device 1, the initialization of a software is performed and the operation of each section is checked (Step S1). The device is brought into a standby state and a standby screen for the standby state is displayed on the display section 8 (Step S2).

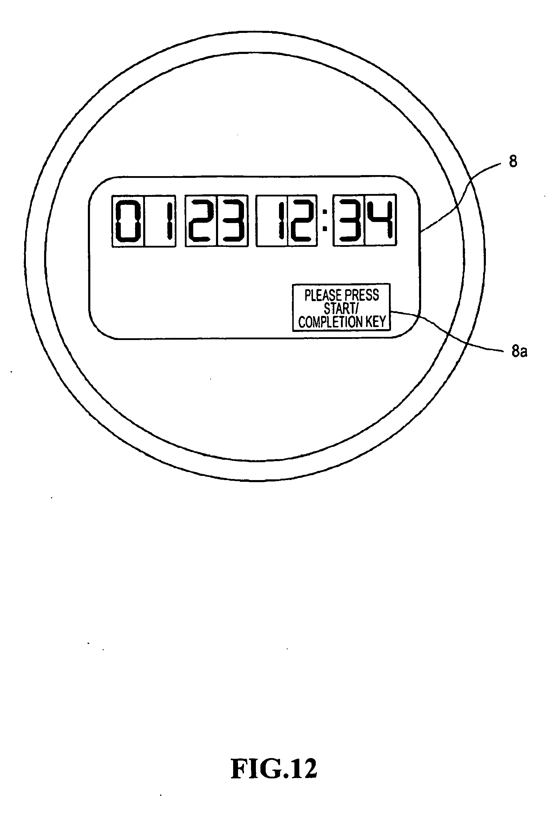

Fig. 12 is a diagram showing an example of the screen displayed when the device 1 is in a standby state. When the device 1 is in a standby state, date and time is displayed at the center of the screen of the display section 8. A lower right area of the screen of the display section 8 functions as a menu display area 8a and displays a message "please press "start/completion" key" to instruct the user to press the "start/completion" key 91a.

Then, in Step S3, the CPU 100a is in a standby state until the "start/completion" key 91a is pressed. When a standby state screen is displayed on the display section 8 and then the user presses the "start/completion" key 91a (Yes in Step S3), the process proceeds to Step S4.

After that, first, the CPU 100a lights the light emitting diodes R1, R2, L1 and L2 provided in the light source section 61 with a predetermined light intensity. The image pick-up area CR (see Fig. 4) is illuminated and a process of imaging the illuminated image pick-up area CR by the image pick-up section 6 is performed. A picked-up image is stored in the frame memory 100e (Step S4).

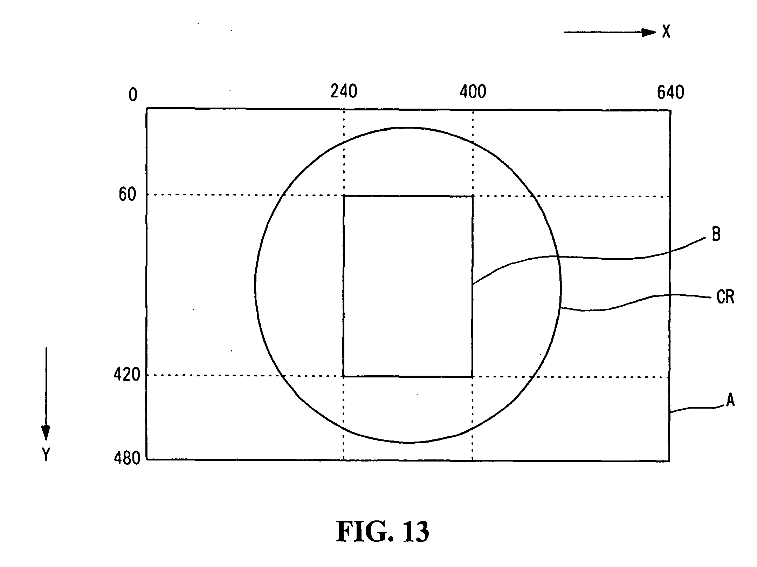

Fig. 13 is a diagram showing the rectangular area including the image pick-up area CR, which is coordinate-divided in a two-dimensional x-y coordinate where $0 \leq x \leq 640$ and $0 \leq y \leq 980$. With a coordinate of the most upper left pixel of a rectangular area A including the image of the image pick-up area CR, which is set to (0,0), the CPU 100a coordinate-divides the area A in the two-dimensional x-y coordinate and selects 4 points of (240, 60), (400, 60), (240, 420) and (400, 420) from the coordinate-divided points. An average luminance of an area B surrounded by the 4 points is obtained (Step S5). The points of the area B obtaining the average luminance are not limited thereto, and needless to say, other coordinates may be used. The area B may have a circular shape or a polygonal shape other than the rectangle.

Next, the CPU 100a determines whether the luminance of the area B is in an objective range or not (Step S6). When the luminance of the area B is beyond the objective range, the light source section input/output interface 100d is used to adjust the current amounts flowing to the light emitting diodes R1, R2, L1 and L2 and adjusts the light intensities of them (Step S7). The process returns to Step S4. When the luminance of the area B is in the objective range (Yes in Step S6), the CPU 100a sets a y coordinate value of a calculation target of a luminance profile to be described later to an initial value (40) (Step S8). In addition, a luminance of the pixel from an end to the other end of the x coordinate of the set y coordinate value (40) is obtained.

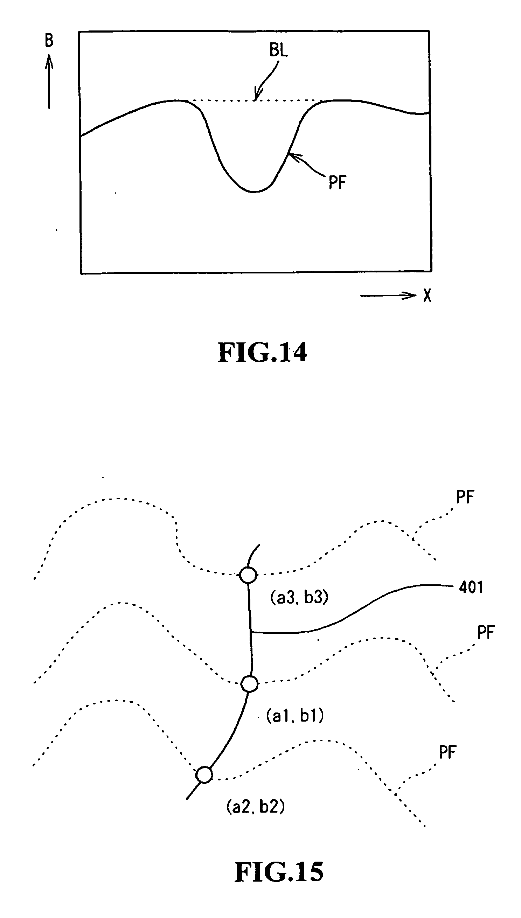

Fig. 14 is a diagram showing an example of the luminance profile (luminance profile PF) of the pixel in an x direction in a predetermined y coordinate. When the luminance is obtained by the above-described process, the luminance profile (luminance profile PF) of the pixel in the x direction in the predetermined y coordinate is obtained (Step S9). Further, the CPU 100a determines whether the set y coordinate value is a final value (440) or not (Step S11). When the y coordinate value is not the final value (440) (No in Step S10), the CPU 100a increments the y coordinate value by a predetermined value (20) (Step S11) and the process returns to Step S10. When the y coordinate value is the final value (440) (Yes in Step S10), the CPU 100a extracts the point having the lowest luminance (hereinafter referred to as "minimum luminance point") in each extracted luminance profile and stores it in the frame memory 100e (Step S12).

Fig. 15 is an explanatory diagram showing a method of obtaining the position of the blood vessel. In order to obtain the position of the blood vessel, the CPU 100a connects a minimum luminance point (a1, b1) near the center of the image of the image pick-up area CR to minimum luminance points (a2, b2) and (a3, b3) adjacent to the minimum luminance point (a1, b1) in a vertical direction. Then, the CPU 100a connects the minimum luminance point (a2, b2) to an adjacent point in the vertical direction and connects the minimum luminance point (a3, b3) to an adjacent point in the vertical direction. The CPU 100a repeats this operation over the entire area of the image to extract the blood vessel as a line array to thereby form a blood vessel pattern 401 (Step S13).

Fig. 16 is a diagram showing an example of the positioning screen of the blood vessel. The CPU 100a causes the display section 8 to display the image of the image pick-up area CR captured in Step S4, the blood vessel pattern 401 formed in Step S5, and the index lines 401a and 401b stored in the flash memory card 100j, as shown in Fig. 16 (Step S14). Moreover, arrows 402a to 402d for instructing the user on the direction in which the position of the image pick-up section 6 should be moved are displayed around the index lines 401a and 401b. In a lower right area of the display section 8, a menu display area 403 is displayed.

Then, the CPU 100a determines whether the blood vessel pattern 401 is positioned in the area 401c (see Fig. 13) or not (Step S15). Herein, the area 401c is an area between the index line 401a on the side of the light emitting diodes L1 and L2 (second light source section) and the index line 401b on the side of the light emitting diodes R1 and R2 (first light source section) of the light source section 61 and is suitable for the image pick-up operation of the CCD image pick-up element 62, as described above. When it is determined that the blood vessel pattern 401 is not positioned in the area 401c (No in Step S15), the CPU 100a allows the process to proceed to Step S16 and performs a process of indicating the direction in which the image pick-up section 6 should be moved by the user.

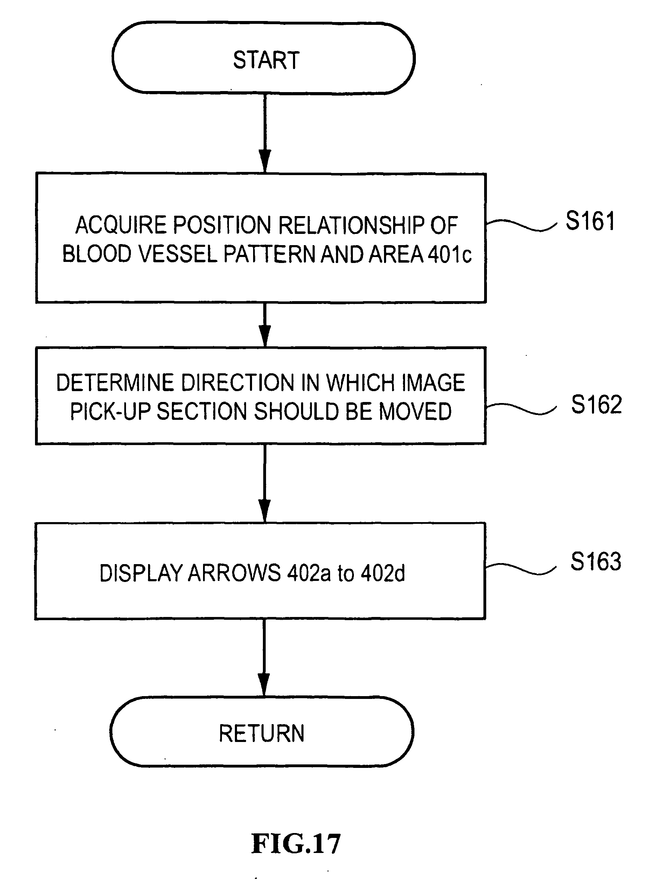

Fig. 17 is a flowchart showing in detail the process of instructing the user of the direction in which the image pick-up section 6 should be moved, which is performed in Step S16 of the flowchart shown in Fig. 11.

When it is determined that the blood vessel pattern 401 is not positioned in the area 401c in the determination process of Step S15, the CPU 100a performs a process of acquiring a position relationship of the blood vessel pattern 401 and the area 401c in Step S161. Herein, the position relationship is information on, for example, whether the blood vessel pattern 401 is positioned in the left side or the right side of the area 401c, the blood vessel pattern 401 is partially positioned in the area 401c or not, the entire blood vessel pattern 401 is accommodated in the area 401c or not if the image pick-up section 6 is rotated, or the like.

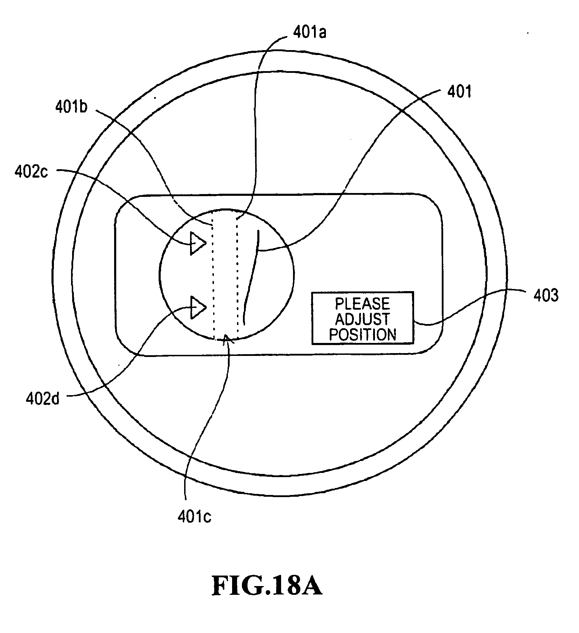

Then, on the basis of the position relationship of the blood vessel pattern 401 and the area 401c acquired in Step S161, the CPU 100a determines the direction in which the image pick-up section 6 should be moved (Step S162) and performs a process of displaying the arrows 402a to 402d for indicating on the display section 8 the direction in which the image pick-up section 6 should be moved (Step S163). Steps S162 and S163 will be described with reference to Figs. 18A to 18C. Figs. 18A to 18C show an example of the display screen of the display section 8 when the movement direction of the image pick-up section is indicated.

For example, when the blood vessel pattern 401 is positioned on the right side of the area 401c as shown in Fig. 18A, first, the CPU 100a determines that the direction in which the image pick-up section 6 should be moved is the "right" in Step S162. Next, in Step S163, the CPU 100a displays on the left side of the area 401c the arrows 402c and 402d for the indication for moving the image pick-up section 6 in the right direction and performs a process of displaying on the menu display area 403 a message for urging the user to perform the position adjustment operation, such as "please adjust the position".

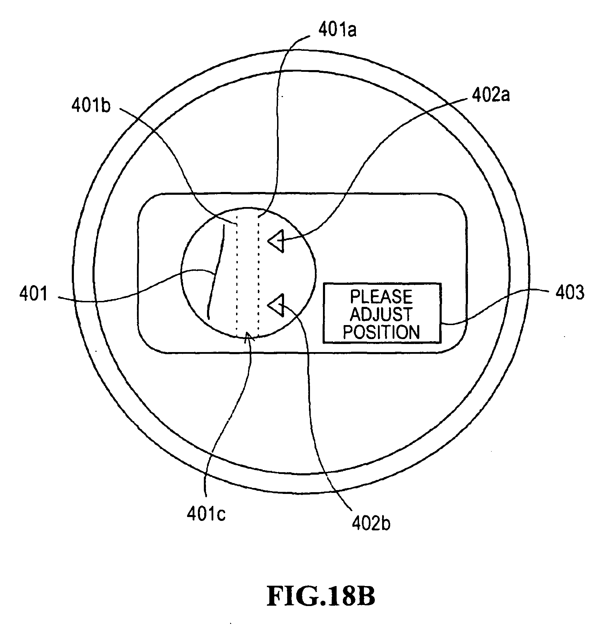

Similarly, when the blood vessel pattern 401 is positioned on the left side of the area 401c as shown in Fig. 18B, first, the CPU 100a determiners that the direction in which the image pick-up section 6 should be moved is the "left" in Step S162. Next, in Step S163, the CPU 100a displays on the right side of the area 401c the arrows 402a and 402b for the indication for moving the image pick-up section 6 in the left direction and performs a process of displaying on the menu display area 403 a message for urging the user to perform the position adjustment operation.

As described above, when the display for the indication for horizontally moving the image pick-up section 6 is displayed, the user moves the image pick-up section 6 by the image pick-up section position adjusting section 7 in accordance with the direction of the arrows 402a to 402d so as to accommodate the blood vessel pattern 401 in the area 401c and adjusts the position.

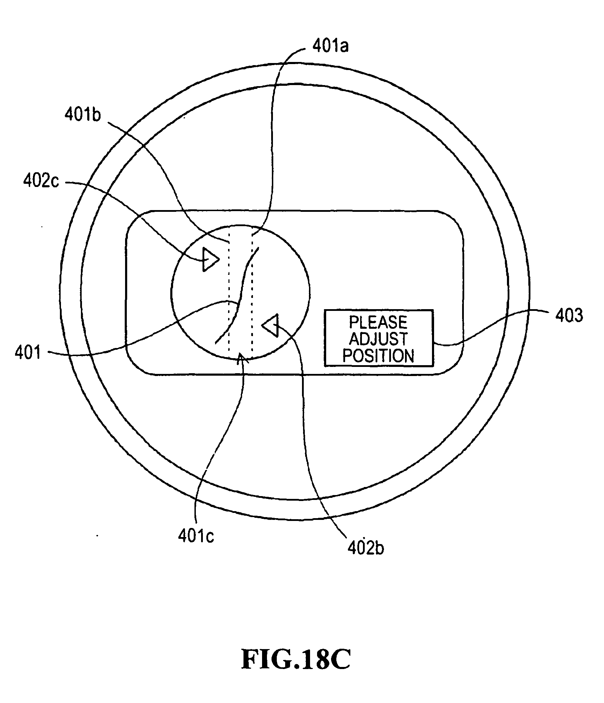

Meanwhile, for example, as shown in Fig. 18C, when a part of the blood vessel pattern 401 is positioned in the area 401c and the entire blood vessel pattern 401 is to be accommodated in the area 401c if the image pick-up section 6 is rotated in a clockwise direction, the CPU 100a determines that the direction in which the image pick-up section 6 should be moved is the "clockwise" in Step S162. Next, in Step S163, the CPU 100a performs a process of displaying in an upper left direction and a lower right direction of the area 401c the arrows 402b and 402c for the indication for rotating the image pick-up section 6 in the clockwise direction.

In this manner, when the display for the indication for rotating the image pick-up section 6 is displayed, the user rotates the image pick-up section 6 in the clockwise direction or a counterclockwise direction by the image pick-up section rotating section 55 in accordance with the direction of the arrows 402a to 402d so as to accommodate the blood vessel pattern 401 in the area 401c and adjusts the image pick-up direction of the image pick-up section 6.

When the direction in which the image pick-up section 6 should be moved is displayed in Step S163, the CPU 100a allows the process to return to the main routine.

When Step S16 is ended, the CPU 100a allows the process to return to Step S4. The CPU 100a captures a picked-up image of the image pick-up area CR again and performs the processes of Steps S4 to S15. The process ranging from the capturing of the picked-up image of the image pick-up area CR in Step S4 to the determination in Step S15 is performed for 1/100 seconds and the display of the display section 8 is also updated for 1/100 seconds. These processes are repeatedly performed even as the user adjusts the position of the image pick-up section 6, and thus the user adjusts the position of the image pick-up section 6 while confirming the display of the display section 8 which is updated as needed. The position adjustment operation is performed by the user and the processes of Steps S4 to S16 are repeated until it is determined that the blood vessel pattern 401 is positioned in the area 401c in the determination process of Step S15.

When, as a result of the position adjustment operation performed by the user, it is determined that the blood vessel pattern 401 is positioned in the area 401c in Step S15 (Yes in Step S15), the CPU 100a activates the "start/completion" key 91a to continue the measurement (Step S17). At this time, the CPU 100a allows all the arrows 402a to 402d to flash as shown in Fig. 16 and displays a message "please press "measurement/record" key" on the menu display area 403, and thus the user is notified of the completion of the position adjustment operation and the activation of the "start/completion" key 91a (Step S18). Next, the CPU 100a determines whether the user has pressed the "measurement/record" key 91b or not (Step S19). Herein, when it is determined that the "measurement/record" key 91b has not been pressed, the CPU 100a allows the process to return to Step S4 to perform the processes of Step S4 to S14 and determines again whether the blood vessel pattern 401 is positioned in the area 401c or not in Step S15. Thanks to this configuration, for example, even when the image pick-up section 6 is not positioned in the area suitable for the image pick-up operation by some kind of operation although the position adjustment operation is performed so as to position the blood vessel pattern 401 in the area 401c, it is possible to instruct the user to perform the position adjustment operation again without continuing the measurement as it is.

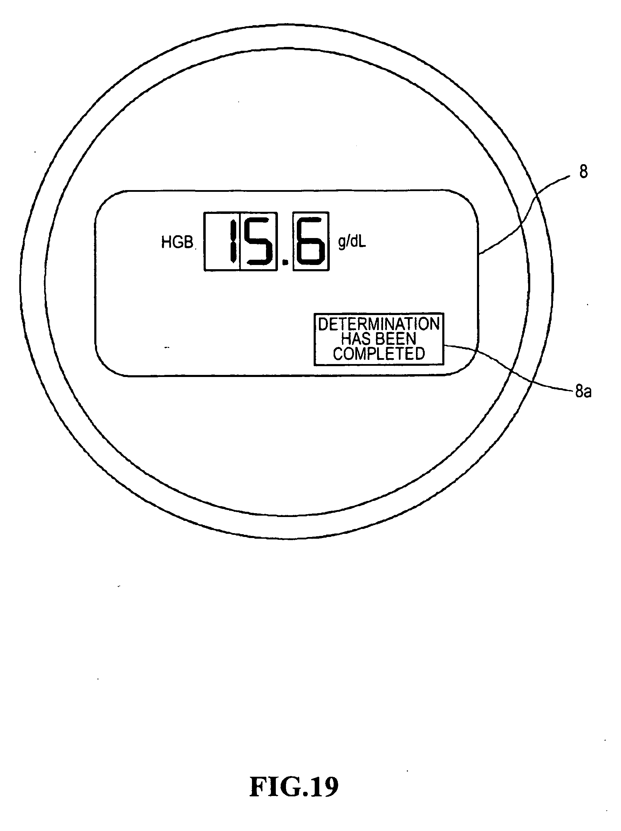

In Step S19, when it is determined that the "measurement/record" key 91b is pressed (Yes in Step S19), the CPU 100a measures hemoglobin concentration (Step S20). After the measurement, a measurement result display screen is displayed on the display section 8, as shown in Fig. 19 (Step S21).

Fig. 19 is a diagram showing an example of the screen when the device 1 completes the measurement. When the measurement result of the concentration of the hemoglobin as a blood component is "15.6 g/dl", the display is displayed on the display section 8 in a digital manner so that the user can see easily. At this time, the menu display area 403 displays a massage "The measurement was completed" and the user is notified of the completion of a set of measurement processes.

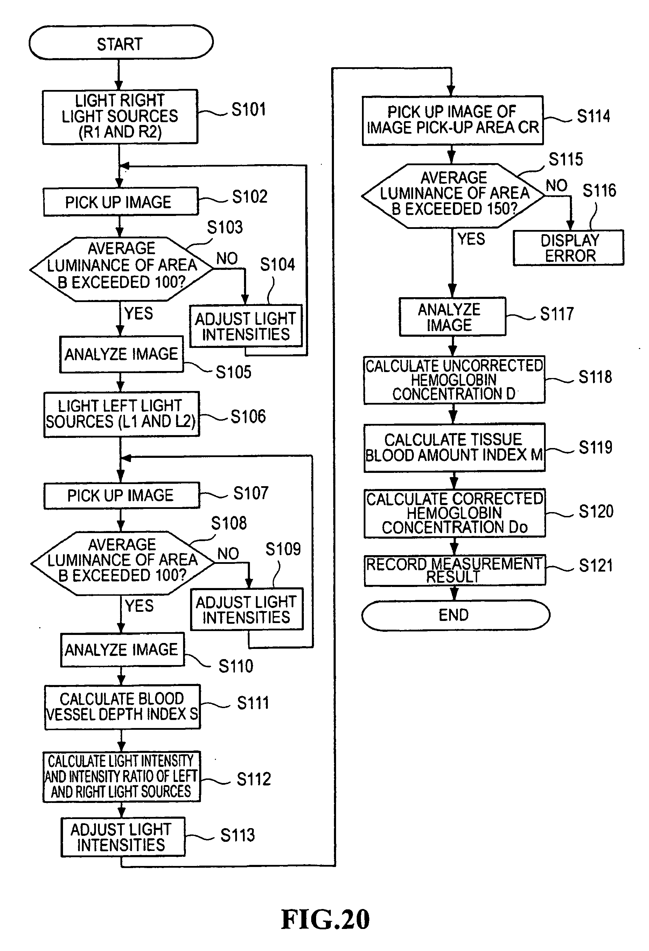

Fig. 20 is a flowchart showing in detail the hemoglobin concentration measuring process performed in Step S20 of the flowchart shown in Fig. 11. When the "measurement/record" key 91b is operated, the CPU 100a controls the light source section input/output interface 100d to illuminate with a proper light intensity the living body including a blood vessel by the light emitting diodes R1 and R2 (first light source section) which are one light source among the light sources disposed on both sides with the blood vessel interposed therebetween (Step S101) and images the illuminated site by the CCD image pick-up element 62 (Step S102). Further, the CPU 100a determines whether the average luminance of the area B exceeds 100 or not (Step S103), and when the luminance does not exceed 100, the CPU uses the light source section input/output interface 100d to adjust the current amounts flowing to the light emitting diodes R1 and R2 to thereby adjust the light intensities of them (Step S104) and the process returns to Step S102.

Herein, in this embodiment, the value of the luminance is a digital converted value (varying from 0 to 255) of an 8-bit A/D converter of the used image input interface 100f. This is because, since the luminance of the image has a proportional relationship with a size of the image signal input from a CCD camera 52c, the A/D converted value of the image signal (0~255) is set as the value of the luminance.

When the average luminance of the area B exceeds 100 (Yes in Step S103), the CPU 100a obtains a luminance profile PF1 and a concentration profile NP1 independent from an incident light intensity about the image obtained in Step S102 (Step S105). Further, the CPU 100a controls the light source section input/output interface 100d to illuminate with a proper light intensity the living body including a blood vessel by the light emitting diodes L1 and L2 (second light source section) which are the other light source among the light sources disposed on both sides with the blood vessel interposed therebetween (Step S106) and images the illuminated site by the CCD image pick-up element 62 (Step S107). Further, the CPU 100a determines whether the average luminance of the area B exceeds 100 or not (Step S108), and when the luminance does not exceed 100, the CPU uses the light source section input/output interface 100d to increase the current amounts flowing to the light emitting diodes L1 and L2 to thereby adjust the light intensity of them (Step S109) and the process returns to Step S107.

When the average luminance of the area B exceeds 100 (Yes in Step S108), the CPU 100a subjects the image obtained in Step S107 to the same process as Step S105 and obtains a luminance profile PF2 and a concentration profile NP2 independent from an incident light intensity (Step S110).

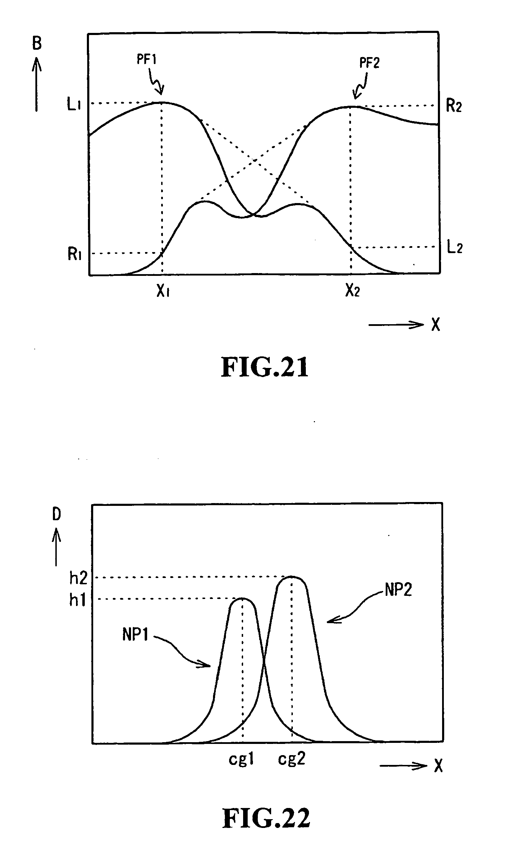

Fig. 21 is a diagram showing the distribution of a luminance B at a position X and the luminance profile PF1 is formed by Step S105 and the luminance profile PF2 is formed by Step S110. Fig. 22 is a diagram showing the distribution of a concentration D at a position X and the concentration profile NP1 is formed by Step S105 and the concentration profile NP2 is formed by Step S110.

The CPU 100a calculates a peak height h1 and a center of gravity coordinate cg1 from the concentration profile NP1 obtained by Step S105 and calculates a peak height h2 and a center of gravity coordinate cg2 from the concentration profile NP2 obtained by Step S110, respectively. Using the calculated values, the CPU calculates a blood vessel depth index S by the following equation (1). The CPU 100a stores the calculation result in the frame memory 100e (Step S111).

$$S = (cg2 - cg1) / \{(h1 + h2)/2\} \ \dots \ (1)$$

The CPU 100a also calculates the light intensity and the intensity ratio of the light sources (light emitting diodes R1 and R2 and light emitting diodes L1 and L2) on the right and left of the blood vessel on the basis of the luminance profile PF1 obtained by Step S105 and the luminance profile PF2 obtained by Step S110 (Step S112). Then, the CPU adjusts the light intensity of both the light sources on the basis of the obtained result (Step S113).

Subsequently, the CPU 100a controls the light source section input/output interface 100d to illuminate the image pick-up area CR (see Fig. 13) by the intensity adjusted light emitting diodes R1, R2, L1 and L2 to thereby image the illuminated area by the CCD image pick-up element 62 (Step S114). Next, the CPU 100a obtains the average luminance of the area B shown in Fig. 13 and determines whether the average luminance of the area B exceeds 150 or not (Step S115). When the luminance does not exceed 150, an error message is displayed (Step S116).

When the average luminance of the area B exceeds 150 (Yes in Step S115), the CPU 100a creates a luminance profile (distribution of luminance B at position X) PF showing a first luminance distribution (Fig. 14) on the axis AX in the image pick-up area CR (see Fig. 13) and reduces a noise component by using a method such as high speed Fourier transform or the like. The CPU 100a then standardizes the luminance profile PF by a baseline BL. The baseline BL is obtained on the basis of the shape of the luminance profile of the part absorbed by the blood vessel. Thus, a concentration profile (distribution of concentrations D at position X) NP independent from an incident light intensity can be obtained (Step S117).

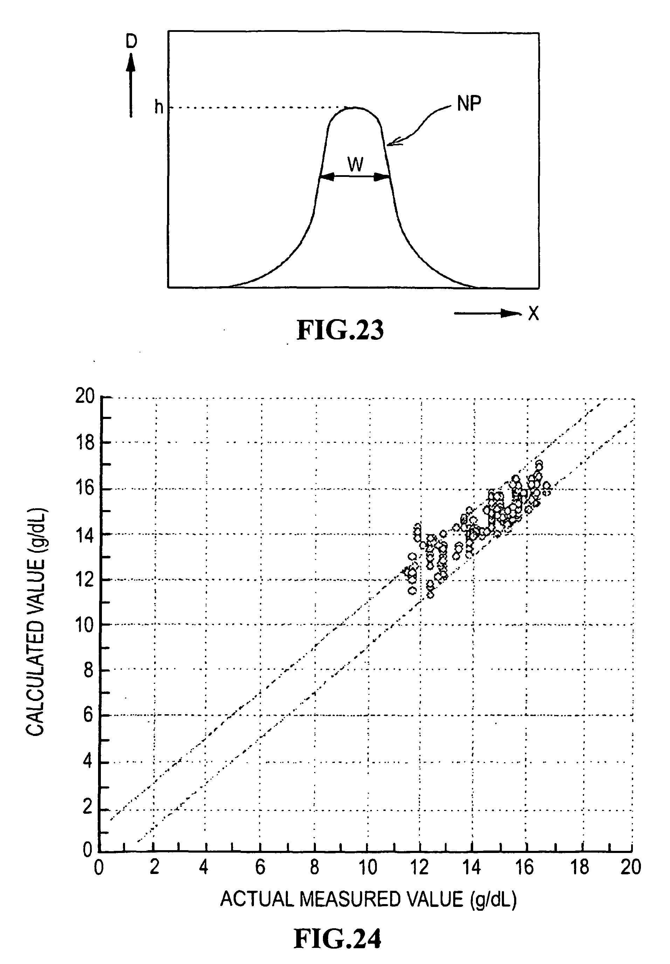

Fig. 23 is a diagram showing the distribution of the concentration D at the position X and the concentration profile NP is formed as shown in the drawing. The CPU 100a then calculates a peak height h and a half-value width w as a distribution width corresponding to a blood vessel diameter on the basis of the created concentration profile NP. The half-value width w is a distribution width of 50% of the peak height of the concentration profile NP. Herein, the obtained h indicates a light intensity ratio of the light absorbed by the measurement target blood vessel (blood) and the light passing through the tissue part thereof, and w indicates a length equivalent to the diameter of the blood vessel in a direction perpendicular to the image pick-up direction. The CPU 100a calculates the uncorrected hemoglobin concentration D by the following equation (2) and stores the result in the frame memory 100e (Step S18).

$$D = h/w^n \ \dots \ (2)$$

Where n is a constant indicating the nonlinear expanse of the half-value width caused by scattering. When there is no light scattering, n=1; and when there is light scattering, n>1.

Next, on the basis of a blood vessel surrounding tissue image in the living body image obtained in Step S101, the CPU 100a calculates a tissue blood amount index M indicating the amount of blood contained in the adjacent tissue. Specifically, a second luminance distribution distributed along the blood vessel image is extracted on the basis of the blood vessel surrounding tissue image in the living body image disposed at a predetermined distance (for example, 2.5 mm) from the blood vessel image in the living body image. Not only the target blood vessel but also the tissue surrounding the blood vessel is imaged in the living body image. Since the light attenuates in proportion to the amount of blood in the tissue, the amount of blood in the surrounding tissue can be estimated by calculating the light attenuation rate of the surrounding tissue. The CPU 100a stores the measurement result in the frame memory 100e (Step S119).

The CPU 100a calculates a correction coefficient fs on the basis of the blood vessel depth index S calculated in Step S111 and a correction coefficient fm on the basis of the tissue blood amount index M calculated in Step S120. Then, using the coefficients, a corrected hemoglobin concentration Do is calculated by the following equation (3) (Step S121).

$$Do = D \times fs \times fm \ \dots \ (3)$$

The CPU 100a stores the calculation result in Step S121 in the frame memory 100e (Step S122) and the process returns to the main routine.

Fig. 24 is a graph plotting the calculated values of the device 1 according to this embodiment and actual measured values obtained from a hemocytometer or the like for the hemoglobin concentrations of a plurality of subjects. As shown in Fig. 24, the actual measured values and the calculated values of the device 1 exist near a straight line having an inclination of 1 and it is understood that the device 1 can measure the hemoglobin concentration with a high degree of accuracy since there is no discrepancy between the actual measured values and the calculated values.

In this embodiment, the description has been made for the noninvasive living body measuring device for measuring the hemoglobin, but is not limited thereto. Various changes can be made if the device measures biological information on the basis of the image obtained by imaging a site of the wrist. For example, the device may measure a blood speed by continuously picking up living body images.

In addition, in this embodiment, the configuration has been employed in which the receiving section 5 is rotated by the image pick-up section position adjusting section 7, but is not limited thereto, and various configurations can be employed. For example, the receiving section 5 may be pivotally connected to the first fixing member 25. Otherwise, the receiving section 5 may include a female-type member and a male-type member which can be slidably inserted into the female-type member and may be configured so as to be stretchable.

Further, in this embodiment, the configuration has been employed in which the receiving section 5 is connected to the first fixing member 25 via the image pick-up section position adjusting section 7, but is not limited thereto. The receiving section 5 may be connected to the device body 2 via the image pick-up section position adjusting section 7.

Further, in this embodiment, the configuration has been employed in which only the second fixing member 43 is provided with the separation preventing member 11, but is not limited thereto. The first fixing member 25 may be provided with the separation preventing member 11 or both of the first and second fixing members 25 and 43 may be provided with the separation preventing member 11.

Further, in this embodiment, the configuration has been employed in which on the positioning screen of the image pick-up section 6 (see Fig. 16), the index lines 401a and 401b are displayed by the display section 8, but is not limited thereto. For example, index members corresponding to the index lines 401a and 401b may be provided on the display screen.

Moreover, in this embodiment, the configuration has been employed in which the CPU 100a automatically repeats the processes of Steps S4 to S16 every predetermined period of time (1/100 second interval) until it is determined that the blood vessel pattern 401 is positioned in the area 401c in the determination process of Step S15 of Fig. 11, but is not limited thereto. For example, the following configuration may be employed.

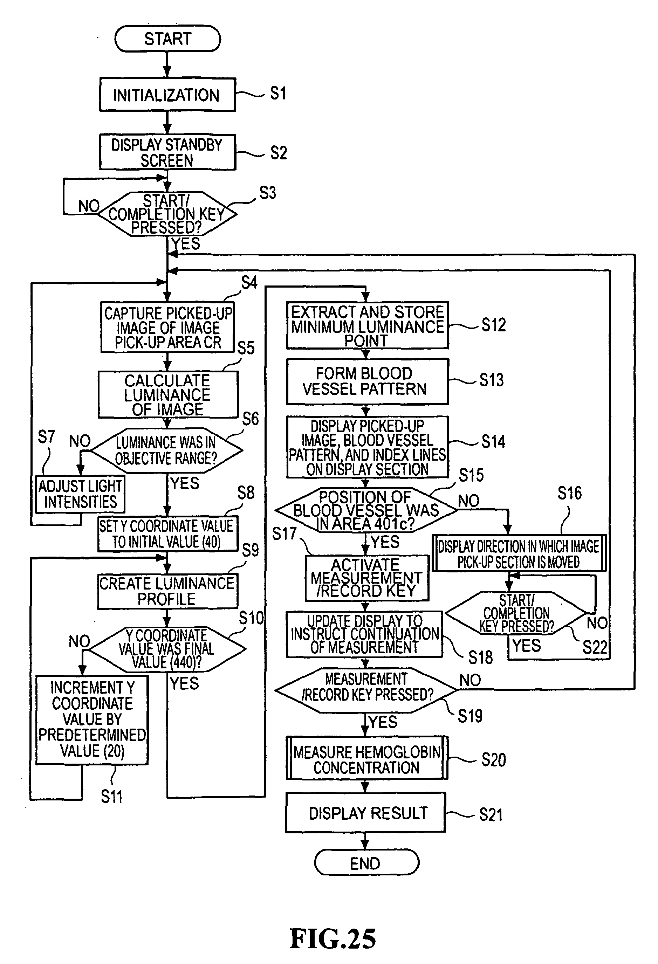

Fig. 25 is a flowchart showing another example of the measurement operation of the device 1. For example, when it is determined that the blood vessel pattern 401 is not positioned in the area 401c in the determination process of Step S15 (No in Step S15), the direction in which the image pick-up section 6 is moved is instructed in Step S16. At this time, the menu display area 403 displays, for example, a message "please adjust the position and press "start/completion" key". Subsequently, in Step S22, the CPU 100a performs a process of determining whether the "start/completion" key 91a is pressed or not. When the "start/completion" key 91a is not pressed (No in Step S22), the process returns and the determination process of Step S22 is repeated until the "start/completion" key 91a is pressed.

The user moves or rotates the image pick-up section 6 in accordance with the display of the arrows 402a to 402d displayed on the display section 8 in Step S16 to adjust the position of the image pick-up section 6 so as to position the blood vessel pattern 401 in the area 401c. When determining that the position adjustment operation is completed, the user presses the "start/completion" key 91a. When the user presses the "start/completion" key 91a (Yes in Step S22), the CPU 100a allows the process to return to Step S4 and performs the processes of Steps S4 to S15 again. In Step S15, it is determined whether the blood vessel pattern 401 is positioned in the area 401c or not, and when it is not determined that the blood vessel pattern 401 is positioned in the area 401c, the CPU 100a allows the process to proceed to Step S16 again and performs a process of instructing the direction in which the image pick-up section 6 is moved. The detailed process of Step S16 is performed in the same manner as that described with reference to Figs. 17 and 18. The process is repeated until it is determined that the blood vessel pattern 401 is positioned in the area 401c.

According to this configuration, after adjusting the position of the image pick-up section 6, the user can know whether the blood vessel pattern 401 is positioned in the area 401c or not at an arbitrary timing. In addition, the device 1 is not necessary to continuously capture and analyze the picked-up image and thus it is preferred in view of saving power consumption.

## Claims

1. A noninvasive living body measuring device comprising:

    an image pick-up section (6) for imaging a living body;
    a device body (2) comprising an analysis section acquiring biological information by analyzing an image obtained by an image pick-up operation of the image pick-up section (6);
    a fixing section (25, 43) for fixing the device body (2) to a living body; and
    an image pick-up section position adjusting section (7) for adjusting a position of the image pick-up section (6) on a living body in a state in which the device body (2) is fixed to a living body,

    **characterized in that**
    the image pick-up section position adjusting section (7) adjusts the position of the image pick-up section (6) relative to the device body (2) by a rotational movement of the image pick-up section (6) relative to the device body (2).

2. The noninvasive living body measuring device of claim 1, further comprising a receiving section (5) for receiving the image pick-up section (6),
    wherein the receiving section (5) is connected to the fixing section (25, 43) or the device body (2) via the image pick-up section position adjusting section (7).

3. The noninvasive living body measuring device of claim 2,
    wherein the image pick-up section position adjusting section (7) rotatably connects the receiving section (5) to the fixing section (25) or the device body (2).

4. The noninvasive living body measuring device of claim 2 or 3, further comprising an image pick-up section rotating section (55) for rotating the image pick-up section (6) in the receiving section (5).

5. The noninvasive living body measuring device of any one of claims 1 to 4, further comprising a display section (8) for displaying a living body image picked up by the image pick-up section (6),
wherein an index indicating an area suitable for the image pick-up operation of the image pick-up section (6) is displayed on the display section (8) along with the living body image, or the living body image is displayed on the display section (8) and an index member indicating the area suitable for the image pick-up operation of the image pick-up section (6) is provided on a screen of the display section (8).

6. The noninvasive living body measuring device of claim 5,
wherein the device body (2) comprises a surface side and a rear side,
the display section (8) is arranged on the surface side of the device body, and
the fixing section (25, 43) is arranged on the rear side of the device body.

7. The noninvasive living body measuring device of claim 2, wherein the fixing section (25, 43) further comprises:

a first fixing member (25) for holding an arm; and
a second fixing member (43) disposed away from the first fixing member at a predetermined interval.

8. The noninvasive living body measuring device of claim 7,
wherein the second fixing member (43) is slidably supported with respect to the device body (2), and
wherein the interval between the first fixing member (25) and the second fixing member (43) is adjustable.

9. The noninvasive living body measuring device of claim 8,
wherein at least one of the first fixing member (25) and the second fixing member (43) is biased in a direction in which the interval therebetween is narrowed.

10. The noninvasive living body measuring device of any one of claims 7 to 9,
wherein at least one of the first fixing member (25) and the second fixing member (43) further comprises:

a separation preventing member (11) for preventing an arm held by the first fixing member (25) and the second fixing member (43) from being separated.

11. The noninvasive living body measuring device of any one claims 7 to 10,
wherein the fixing section is configured so as to hold an arm by the first fixing member (25) and the second fixing member (43) from palmar side and back side of a hand.

12. The noninvasive living body measuring device of any one of claims 1 to 11, further comprising:

position relationship acquiring means for acquiring a relationship between an area suitable for the image pick-up operation of the image pick-up section (6) and the position of the image pick-up section (6); and
indication means (402) for indicating the direction in which the image pick-up section (6) should be moved on the basis of the relationship between the area and the position of the image pick-up section (6) acquired by the position relationship acquiring means.

13. The noninvasive living body measuring device of claim 12,
wherein the position relationship acquiring means acquires the relationship between the area and the position of the image pick-up section (6) each time a predetermined period of time elapses, and
wherein the indication means (402) updates the direction in which the image pick-up section (6) should be moved each time the position relationship acquiring means acquires the relationship between the area and the position of the image pick-up section (6).

14. The noninvasive living body measuring device of claim 12, further comprising acquisition instructing means for instructing the position relationship acquiring means to acquire the relationship between the area and the position of the image pick-up section (6) if a blood vessel pattern (401) is not positioned in the area (401c) suitable for the image pick-up operation,
wherein the position relationship acquiring means acquires the relationship between the area and the position of the image pick-up section (6) when the acquisition operation is instructed by the acquisition instructing means, and

wherein the indication means (402) updates the direction in which the image pick-up section (6) should be moved each time the position relationship acquiring means acquires the relationship between the area and the position of the image pick-up section (6).

15. A noninvasive living body measuring method comprising the steps of:

fixing to an arm a device body (2) including an image pick-up section (6) for imaging a living body and an analysis section for acquiring biological information by analyzing an image obtained by the image pick-up operation of the image pick-up section (6);
adjusting a position of the image pick-up section (6) on palmar side of a wrist of the arm relative to the device body (2) by a rotational movement of the image pick-up section (6) relative to the device body (2);
imaging a site of the living body corresponding to the position adjusted in the adjusting step by the image pick-up section (6); and
acquiring a biological information by analyzing with the analysis section an image obtained in the imaging step.

**Patentansprüche**

1. Vorrichtung zur nichtinvasiven Messung eines lebenden Körpers, mit:

einem Bildaufnahmeabschnitt (6) zum Abbilden eines lebenden Körpers;
einem Vorrichtungskörper (2) mit einem Analyseabschnitt zum Erfassen biologischer Information durch Analysieren eines durch eine Bildaufnahmeoperation des Bildaufnahmeabschnitts (6) erhaltenden Bildes;
einem Fixierungsabschnitt (25, 43) zum Fixieren des Vorrichtungskörpers (2) an einem lebenden Körper; und
einem Bildaufnahmeabschnitt-Positionsanpassungsabschnitt (7) zum Anpassen einer Position des Bildaufnahmeabschnitts (6) an einem lebenden Körper in einem Zustand, bei dem der Vorrichtungskörper (2) an einem lebenden Körper fixiert ist,

**dadurch gekennzeichnet, dass**
der Bildaufnahmeabschnitt-Positionsanpassungsabschnitt (7) die Position des Bildaufnahmeabschnitts (6) relativ zu dem Vorrichtungskörpers (2) durch eine Drehbewegung des Bildaufnahmeabschnitts (6) relativ zu dem Vorrichtungskörper (2) anpasst.

2. Vorrichtung zur nichtinvasiven Messung eines lebenden Körpers nach Anspruch 1, ferner mit einem Aufnahmeabschnitt (5) zur Aufnahme des Bildaufnahmeabschnitts (6),
wobei der Aufnahmeabschnitt (5) mit dem Fixierungsabschnitt (25, 43) oder dem Vorrichtungskörper (2) über den Bildaufnahmeabschnitt-Positionsanpassungsabschnitt (7) verbunden ist.

3. Vorrichtung zur nichtinvasiven Messung eines lebenden Körpers nach Anspruch 2,
wobei der Bildaufnahmeabschnitt-Positionsanpassungsabschnitt (7) den Aufnahmeabschnitt (5) mit dem Fixierungsabschnitt (25) oder dem Vorrichtungskörper (2) rotierbar verbindet.

4. Vorrichtung zur nichtinvasiven Messung eines lebenden Körpers nach Anspruch 2 oder 3, ferner mit einem Bildaufnahmeabschnitt-Rotierabschnitt (55) zum Rotieren des Bildaufnahmeabschnitts (6) in dem Aufnahmeabschnitt (5).

5. Vorrichtung zur nichtinvasiven Messung eines lebenden Körpers nach Anspruch 1 bis 4, ferner mit einem Anzeigeabschnitt (8) zum Anzeigen eines Bildes eines lebenden Körpers, das durch den Bildaufnahmeabschnitts (6) aufgenommen wird,
wobei ein Index, der einen Bereich anzeigt, der für die Bildaufnahmeoperation des Bildaufnahmeabschnitts (6) geeignet ist, auf dem Anzeigeabschnitt (8) zusammen mit dem Bild eines lebenden Körpers angezeigt wird, oder das Bild eines lebenden Körpers auf dem Anzeigeabschnitt (8) angezeigt wird und ein Indexelement, das den Bereich anzeigt, der für die Bildaufnahmeoperation des Bildaufnahmeabschnitts (6) geeignet ist, auf einem Bildschirm des Anzeigeabschnitt (8) bereitgestellt wird.

6. Vorrichtung zur nichtinvasiven Messung eines lebenden Körpers nach Anspruch 5,
wobei der Vorrichtungskörper (2) eine Oberflächenseite und eine Rückseite umfasst,
der Anzeigenabschnitt (8) auf der Oberflächenseite des Vorrichtungskörpers angeordnet ist, und

der Fixierungsabschnitt (25, 43) auf der Rückseite des Vorrichtungskörpers angeordnet ist.

7. Vorrichtung zur nichtinvasiven Messung eines lebenden Körpers nach Anspruch 2,
wobei der Fixierungsabschnitt (25, 43) ferner umfasst:

ein erstes Fixierungselement (25) zum Fixieren eines Armes; und
ein zweites Fixierungselement (43), das von dem ersten Fixierungselement (25) in einem vorgegebenen Intervall entfernt angeordnet ist.

8. Vorrichtung zur nichtinvasiven Messung eines lebenden Körpers nach Anspruch 7,
wobei das zweite Fixierungselement (43) bezüglich des Vorrichtungskörpers (2) verschiebbar unterstützt wird, und wobei das Intervall zwischen dem ersten Fixierungselement (25) und dem zweiten Fixierungselement (43) anpassbar ist.

9. Vorrichtung zur nichtinvasiven Messung eines lebenden Körpers nach Anspruch 8,
wobei zumindest eines von dem ersten Fixierungselement (25) und dem zweiten Fixierungselement (43) in einer Richtung ausgerichtet ist, in der das dazwischenliegende Intervall verengt ist.

10. Vorrichtung zur nichtinvasiven Messung eines lebenden Körpers nach irgendeinem der Ansprüche 7 bis 9,
wobei zumindest eines von dem ersten Fixierungselement (25) und dem zweiten Fixierungselement (43) ferner umfasst:

ein Separationsverhinderungselement (11) zum Verhindern, dass ein Arm, der durch das erste Fixierungselement (25) und durch das zweite Fixierungselement (43) gehalten wird, separiert wird.

11. Vorrichtung zur nichtinvasiven Messung eines lebenden Körpers nach irgendeinem der Ansprüche 7 bis 10,
wobei der Fixierungsabschnitt konfiguriert ist, einen Arm durch das erste Fixierungselement (25) und durch das zweite Fixierungselement (43) von einer Handflächenseite und einer Rückseite einer Hand zu halten.

12. Vorrichtung zur nichtinvasiven Messung eines lebenden Körpers nach irgendeinem der Ansprüche 1 bis 11, ferner mit:

einem Positionsbeziehungs-Erfassungsmittel zum Erfassen einer Beziehung zwischen einem Bereich, der für die Bildaufnahmeoperation des Bildaufnahmeabschnitts (6) geeignet ist, und der Position des Bildaufnahmeabschnitts (6); und
einem Anzeigemittel (402) zum Anzeigen der Richtung, in die der Bildaufnahmeabschnitts (6) auf Grundlage der Beziehung zwischen dem Bereich und der Position des Bildaufnahmeabschnitts (6), die durch das Positionsbeziehungs-Erfassungsmittel erfasst wird, bewegt werden soll.

13. Vorrichtung zur nichtinvasiven Messung eines lebenden Körpers nach Anspruch 12,
wobei das Positionsbeziehungs-Erfassungsmittel die Beziehung zwischen dem Bereich und der Position des Bildaufnahmeabschnitts (6) bei jedem Mal erfasst, wenn eine vorgegebene Zeitperiode abläuft, und
wobei das Anzeigemittel (402) die Richtung aktualisiert, in die der Bildaufnahmeabschnitt (6) bewegt werden soll, bei jedem Mal, wenn das Positionsbeziehungs-Erfassungsmittel die Beziehung zwischen dem Bereich und der Position des Bildaufnahmeabschnitts (6) erfasst.

14. Vorrichtung zur nichtinvasiven Messung eines lebenden Körpers nach Anspruch 12, ferner mit einem Erfassungsanweisungsmittel zum Anweisen des Positionsbeziehungs-Erfassungsmittels, die Beziehung zwischen dem Bereich und der Position des Bildaufnahmeabschnitts (6) zu erfassen, wenn ein Blutgefäßmuster (401) nicht in dem Bereich (401c) angeordnet ist, der für die Bildaufnahmeoperation geeignet ist,
wobei das Positionsbeziehungs-Erfassungsmittel die Beziehung zwischen dem Bereich und der Position des Bildaufnahmeabschnitts (6) erfasst, wenn die Erfassungsoperation durch das Erfassungsanweisungsmittel angewiesen wird, und
wobei das Anzeigemittel (402) die Richtung, in die der Bildaufnahmeabschnitt (6) bewegt werden soll, jedes Mal dann aktualisiert, wenn das Positionsbeziehungs-Erfassungsmittel die Beziehung zwischen dem Bereich und der Position des Bildaufnahmeabschnitts (6) erfasst.

15. Verfahren zur nichtinvasiven Messung eines lebenden Körpers, mit den Schritten:

Fixieren an einem Arm eines Vorrichtungskörpers (2) mit einem Bildaufnahmeabschnitt (6) zum Abbilden eines lebenden Körpers zum Erfassen biologischer Information durch Analysieren eines Bildes, das durch die Bildaufnahmeoperation des Bildaufnahmeabschnitts (6) erhalten wird;

Anpassen einer Position des Bildaufnahmeabschnitt (6) auf einer Handflächenseite eines Handgelenks des Armes relativ zu dem Vorrichtungskörper (2) durch eine Rotationsbewegung des Bildaufnahmeabschnitts (6) relativ zu dem Vorrichtungskörper (2);

Abbilden einer Seite des lebenden Körpers, die mit der Position zusammenhängt, die in dem Anpassungsschritt durch den Bildaufnahmeabschnitts (6) angepasst wurde; und

Erfassen biologischer Information durch Analysieren eines Bildes, das in dem Abbildungsschritt erhalten wurde, mit dem Analyseabschnitt.

## Revendications

1. Dispositif non-invasif de mesure d'un corps vivant comprenant:

   une section (6) de capture d'image pour former une image d'un corps vivant ;
   un corps de dispositif (2) comprenant une section d'analyse obtenant des informations biologiques en analysant une image obtenue par une opération de capture d'image de la section (6) de capture d'image ;
   une section (25, 43) de fixation pour fixer le corps de dispositif (2) à un corps vivant ; et
   une section (7) d'ajustement de position de la section de capture d'image pour ajuster une position de la section (6) de capture d'image sur un corps vivant dans un état où le corps de dispositif (2) est fixé à un corps vivant,

   **caractérisé en ce que**
   la section (7) d'ajustement de position de la section de capture d'image ajuste la position de la section (6) de capture d'image par rapport au corps de dispositif (2) par un mouvement de rotation de la section (6) de capture d'image par rapport au corps de dispositif (2).

2. Dispositif non-invasif de mesure d'un corps vivant de la revendication 1, comprenant en outre une section (5) de réception pour recevoir la section (6) de capture d'image,
   où la section (5) de réception est reliée à la section (25, 43) de fixation ou au corps de dispositif (2) à travers la section (7) d'ajustement de position de la section de capture d'image.

3. Dispositif non-invasif de mesure d'un corps vivant de la revendication 2,
   dans lequel la section (7) d'ajustement de position de la section de capture d'image relie en rotation la section (5) de réception à la section (25) de fixation ou au corps de dispositif (2).

4. Dispositif non-invasif de mesure d'un corps vivant de la revendication 2 ou 3, comprenant en outre une section (55) rotative de la section de capture d'image pour mettre en rotation la section (6) de capture d'image dans la section (5) de réception.

5. Dispositif non-invasif de mesure d'un corps vivant de l'une quelconque des revendications 1 à 4, comprenant en outre une section (8) d'affichage pour afficher une image de corps vivant capturée par la section (6) de capture d'image,
   où un indice indiquant une zone appropriée pour l'opération de capture d'image de la section (6) de capture d'image est affiché sur la section (8) d'affichage avec l'image de corps vivant, ou l'image de corps vivant est affichée sur la section (8) d'affichage et un élément d'indice indiquant la zone appropriée pour l'opération de capture d'image de la section (6) de capture d'image est prévu sur un écran de la section (8) d'affichage.

6. Dispositif non-invasif de mesure d'un corps vivant de la revendication 5,
   dans lequel le corps de dispositif (2) comprend un côté de surface et un côté arrière,
   la section (8) d'affichage est agencée sur le côté de surface du corps de dispositif, et
   la section (25, 43) de fixation est agencée sur le côté arrière du corps de dispositif.

7. Dispositif non-invasif de mesure d'un corps vivant de la revendication 2,
   dans lequel la section (25, 43) de fixation comprend en outre :

   un premier élément de fixation (25) pour maintenir un bras ; et

un deuxième élément de fixation (43) disposé à l'écart du premier élément de fixation à un intervalle prédéterminé.

8. Dispositif non-invasif de mesure d'un corps vivant de la revendication 7,
dans lequel le deuxième élément de fixation (43) est soutenu en coulissement par rapport au corps de dispositif (2), et
dans lequel l'intervalle entre le premier élément de fixation (25) et le deuxième élément de fixation (43) est réglable.

9. Dispositif non-invasif de mesure d'un corps vivant de la revendication 8,
dans lequel au moins l'un du premier élément de fixation (25) et du deuxième élément de fixation (43) est maintenu dans une direction où l'intervalle entre eux est rétréci.

10. Dispositif non-invasif de mesure d'un corps vivant de l'une quelconque des revendications 7 à 9,
dans lequel au moins l'un du premier élément de fixation (25) et du deuxième élément de fixation (43) comprend en outre:

un élément (11) d'empêchement de séparation pour empêcher la séparation d'un bras maintenu par le premier élément de fixation (25) et le deuxième élément de fixation (43).

11. Dispositif non-invasif de mesure d'un corps vivant de l'une quelconque des revendications 7 à 10,
dans lequel la section de fixation est configurée de sorte à maintenir un bras par le premier élément de fixation (25) et le deuxième élément de fixation (43) par la paume et le dos d'une main.

12. Dispositif non-invasif de mesure d'un corps vivant de l'une quelconque des revendications 1 à 11, comprenant en outre :

un moyen d'acquisition de relation de position pour acquérir une relation entre une zone appropriée pour l'opération de capture d'image de la section (6) de capture d'image et la position de la section (6) de capture d'image ; et
un moyen d'indication (402) pour indiquer la direction où la section (6) de capture d'image devrait être déplacée sur la base de la relation entre la zone et la position de la section (6) de capture d'image acquise par le moyen d'acquisition de relation de position.

13. Dispositif non-invasif de mesure d'un corps vivant de la revendication 12,
où le moyen d'acquisition de relation de position acquiert la relation entre la zone et la position de la section (6) de capture d'image à chaque fois qu'une durée prédéterminée s'écoule, et
où le moyen d'indication (402) met à jour la direction dans laquelle la section (6) de capture d'image devrait être déplacée à chaque fois que le moyen d'acquisition de relation de position acquiert la relation entre la zone et la position de la section (6) de capture d'image.

14. Dispositif non-invasif de mesure d'un corps vivant de la revendication 12, comprenant en outre un moyen d'instruction d'acquisition pour donner l'instruction au moyen d'acquisition de relation de position d'acquérir la relation entre la zone et la position de la section (6) de capture d'image si un modèle de vaisseau sanguin (401) n'est pas positionné dans la zone (401c) appropriée à l'opération de capture d'image,
où le moyen d'acquisition de relation de position acquiert la relation entre la zone et la position de la section (6) de capture d'image lorsque le moyen d'instruction d'acquisition donne une instruction à l'opération d'acquisition, et
où le moyen d'indication (402) met à jour la direction dans laquelle la section (6) de capture d'image devrait être déplacée à chaque fois que le moyen d'acquisition de relation de position acquiert la relation entre la zone et la position de la section (6) de capture d'image.

15. Procédé non-invasif de mesure d'un corps vivant comprenant les étapes qui consistent :

à fixer à un bras un corps de dispositif (2) comportant une section (6) de capture d'image pour former une image d'un corps vivant et une section d'analyse pour acquérir des informations biologiques en analysant une image obtenue par l'opération de capture d'image de la section (6) de capture d'image ;
à ajuster une position de la section (6) de capture d'image sur la face palmaire d'un poignet du bras par rapport au corps de dispositif (2) par un mouvement de rotation de la section (6) de capture d'image par rapport au corps de dispositif (2) ;
à former une image d'un site du corps vivant correspondant à la position ajustée lors de l'étape d'ajustement par la section (6) de capture d'image ; et
à obtenir des informations biologiques en analysant, avec la section d'analyse, une image obtenue à l'étape de formation d'image.

**FIG.1**

FIG. 2

**FIG.3**

**FIG.4**

EP 2 074 938 B1

10

100a

100g

CPU

100b — MAIN MEMORY

INPUT INTERFACE

START/ COMPLETION KEY — 91a

MEASUREMENT /RECORD KEY — 91b

100e

100i

100c

1

FRAME MEMORY

IMAGE OUTPUT INTERFACE

DISPLAY SECTION — 8

100d

100j — FLASH MEMORY CARD

FLASH MEMORY CARD READER

LIGHT SOURCE SECTION INPUT /OUTPUT INTERFACE

LIGHT SOURCE SECTION — 61

62

COMMUNICATION INTERFACE

IMAGE INPUT INTERFACE

CCD IMAGE PICK-UP ELEMENT — 62

TO EXTERNAL ACCESS DEVICE

IMAGE PICK-UP SECTION — 60

100h

CONTROL SECTION

NONINVASIVE LIVING BODY MEASURING DEVICE

100f

**FIG. 5**

FIG. 6A     FIG. 6B     FIG. 6C

**FIG.7**

## FIG. 8A

## FIG. 8B

## FIG. 8C

**FIG.9**

**FIG.10**

**FIG.11**

PLEASE PRESS
START/
COMPLETION KEY

8

8a

**FIG.12**

**FIG. 13**

EP 2 074 938 B1

**FIG.14**

**FIG.15**

PLEASE PRESS
MEASUREMENT
/RECORD KEY

**FIG.16**

START

ACQUIRE POSITION RELATIONSHIP OF
BLOOD VESSEL PATTERN AND AREA 401c          S161

DETERMINE DIRECTION IN WHICH IMAGE
PICK-UP SECTION SHOULD BE MOVED            S162

DISPLAY ARROWS 402a to 402d                S163

RETURN

**FIG.17**

401a

401b

402c

401

PLEASE
ADJUST
POSITION

403

402d

401c

# FIG.18A

**FIG.18B**

401a

401b

402c

401

401c

402b

403

PLEASE
ADJUST
POSITION

**FIG.18C**

HGB **15.6** g/dL

8

DETERMINATION
HAS BEEN
COMPLETED

8a

**FIG.19**

START

LIGHT RIGHT
LIGHT SOURCES — S101
(R1 AND R2)

PICK UP IMAGE — S102

S103
AVERAGE
LUMINANCE OF AREA — NO — S104
B EXCEEDED 100?
YES
ADJUST LIGHT
ANALYZE IMAGE INTENSITIES
S105

LIGHT LEFT LIGHT
SOURCES (L1 AND L2) — S106

PICK UP IMAGE — S107

S108
AVERAGE
LUMINANCE OF AREA — NO — S109
B EXCEEDED 100?
YES
ADJUST LIGHT
ANALYZE IMAGE INTENSITIES
S110

CALCULATE BLOOD
VESSEL DEPTH INDEX S — S111

CALCULATE LIGHT INTENSITY
AND INTENSITY RATIO OF LEFT — S112
AND RIGHT LIGHT SOURCES

ADJUST LIGHT
INTENSITIES — S113

PICK UP IMAGE OF
IMAGE PICK-UP AREA CR — S114

S115
AVERAGE
LUMINANCE OF AREA — NO — S116
B EXCEEDED 150?
YES
DISPLAY
ERROR

ANALYZE
IMAGE — S117

CALCULATE UNCORRECTED
HEMOGLOBIN — S118
CONCENTRATION D

CALCULATE TISSUE
BLOOD AMOUNT INDEX M — S119

CALCULATE CORRECTED
HEMOGLOBIN — S120
CONCENTRATION Do

RECORD MEASUREMENT — S121
RESULT

END

**FIG.20**

**FIG.21**

**FIG.22**

**FIG.23**

**FIG.24**

**FIG.25**

**REFERENCES CITED IN THE DESCRIPTION**

**Patent documents cited in the description**

- US 7280860 B
- EP 1743570 A1
- EP 1447044 A1